(19) Europäisches Patentamt
European Patent Office
Office européen des brevets

(11) **EP 1 225 171 A1**

(12) **EUROPÄISCHE PATENTANMELDUNG**

(43) Veröffentlichungstag:
**24.07.2002 Patentblatt 2002/30**

(51) Int Cl.$^7$: **C07D 239/54**, C07D 405/12, A01N 43/54

(21) Anmeldenummer: **02009276.3**

(22) Anmeldetag: **17.06.1996**

(84) Benannte Vertragsstaaten:
**CH DE ES FR GB IT LI**

(30) Priorität: **29.06.1995 DE 19523650**
**19.12.1995 DE 19547475**

(62) Dokumentnummer(n) der früheren Anmeldung(en) nach Art. 76 EPÜ:
**96922004.5 / 0 837 853**

(71) Anmelder:
- **BAYER AG**
  **51368 Leverkusen (DE)**
- **NIHON BAYER AGROCHEM K.K.**
  **Tokyo 108 (JP)**

(72) Erfinder:
- **Andree, Roland, Dr.**
  **40764 Langenfeld (DE)**
- **Drewes, Mark Wilhelm, Dr.**
  **40764 Langenfeld (DE)**
- **Schallner, Otto, Dr.**
  **40789 Monheim (DE)**
- **Dollinger, Markus, Dr.**
  **51381 Leverkusen (DE)**
- **Santel, Hans-Joachim, Dr.**
  **51371 Leverkusen (DE)**
- **Erdelen, Christoph, Dr.**
  **42799 Leichlingen (DE)**
- **Yanagi, Akihiko**
  **Oyama-shi, Tochigi 323-0829 (JP)**
- **Goto, Toshio**
  **Shimotsuga-gun, Tochigi 329-0414 (JP)**

(74) Vertreter: **Krieg, Robert Alexander et al**
**Bayer AG,**
**Konzernbereich RP,**
**Patente und Lizenzen**
**51368 Leverkusen (DE)**

Bemerkungen:
Diese Anmeldung ist am 29 - 04 - 2002 als Teilanmeldung zu der unter INID-Kode 62 erwähnten Anmeldung eingereicht worden.

(54) **Substituierte Cyanophenyluracile**

(57) Die Erfidnung betrifft neue substituierte Cyanophenyluracile der Formel (I)

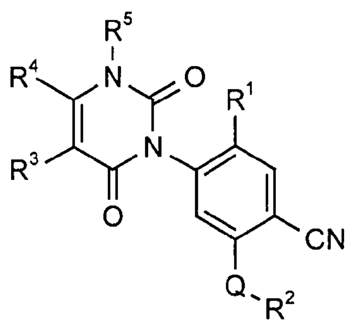

(I)

in welcher

Q für O, S, SO oder $SO_2$ steht,

$R^1$ für Wasserstoff, Cyano, Fluor oder Chlor steht,

EP 1 225 171 A1

$R^2$ für jeweils gegebenenfalls durch Cyano, Carboxy, Carbamoyl, Thiocarbamoyl, Fluor, Chlor, Brom, durch $C_1$-$C_4$-Alkyl, $C_1$-$C_4$-Alkoxy, $C_1$-$C_4$-Alkylthio, $C_1$-$C_4$-Alkylsulfinyl, $C_1$-$C_4$-Alkylsulfonyl, $C_1$-$C_4$-Alkyl-carbonyl oder $C_1$-$C_4$-Alkoxy-carbonyl (welches jeweils gegebenenfalls durch Fluor und/oder Chlor sind), durch Phenyl, Phenoxy oder Phenylthio (welche jeweils gegebenenfalls durch Fluor, Chlor, Brom, Cyano, Methyl, Methoxy, Trifluormethyl und/oder Trifluormethoxy substituiert sind) substituiertes Furyl, Tetrahydrofuryl, Thienyl, Tetrahydrothienyl, Oxetanyl, Thietanyl, Oxazolyl, Isoxazolyl, Thiazolyl, Oxadiazolyl, Thiadiazolyl, Pyrazolyl, Pyridinyl, Pyrimidinyl, Triazinyl, Indolyl, Chinolinyl oder Chinoxalinyl steht,

$R^3$ für Wasserstoff, Fluor, Chlor, Brom oder für jeweils gegebenenfalls durch Fluor und/oder Chlor substituiertes Alkyl oder Alkoxy mit jeweils 1 bis 4 Kohlenstoffatomen steht,

$R^4$ für gegebenenfalls durch Fluor und/oder Chlor substituiertes Alkyl mit 1 bis 4 Kohlenstoffatomen steht und

$R^5$ für Wasserstoff oder für jeweils gegebenenfalls durch Fluor, Chlor oder $C_1$-$C_4$-Alkoxy substituiertes Alkyl, Alkoxy, Alkenyl oder Alkinyl mit jeweils bis zu 6 Kohlenstoffatomen steht,

Verfahren zu ihrer Herstellung sowie ihre Verwendung als Herbizide.

## Beschreibung

Die Erfindung betrifft neue substituierte Cyanophenyluracile, Verfahren zu ihrer Herstellung sowie ihre Verwendung als Pflanzenbehandlungsmittel, insbesondere als Herbizide und Insektizide.

Es ist bekannt, daß bestimmte substituierte Phenyluracile herbizide Eigenschaften aufweisen (vgl. EP-408 382/US-5 084 084/US-5 127 935/US-5 154 755, EP-563 384, EP-648 749, WO 91/00278, US-4 979 982, US-5 169 430). Diese Verbindungen haben jedoch bisher keine nennenswerte Bedeutung erlangt.

Es wurden nun die neuen substituierten Cyanophenyluracile der allgemeinen Formel (I) gefunden

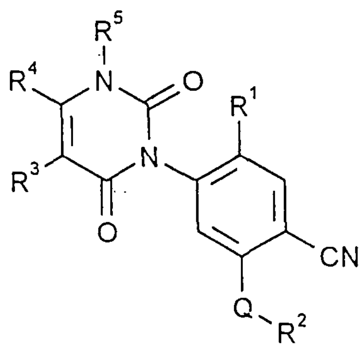

(I)

in welcher

Q für O, S, SO oder $SO_2$ steht,

$R^1$ für Wasserstoff, Cyano oder Halogen steht,

$R^2$ für Wasserstoff oder für einen jeweils gegebenenfalls substituierten Rest der Reihe Alkyl, Alkenyl, Alkinyl, Cycloalkyl, Cycloalkylalkyl, Aryl, Arylalkyl, Heterocyclyl oder Heterocyclylalkyl steht,

$R^3$ für Wasserstoff, Halogen oder für einen jeweils gegebenenfalls substituierten Rest der Reihe Alkyl oder Alkoxy steht,

$R^4$ für gegebenenfalls substituiertes Alkyl steht und

$R^5$ für Wasserstoff oder für einen jeweils gegebenenfalls substituierten Rest der Reihe Alkyl, Alkoxy, Alkenyl oder Alkinyl steht,

wobei die bekannten Verbindungen 1-(4-Cyano-2-fluor-5-methylthio-phenyl)-3,6-dihydro-2,6-dioxo-3-methyl-4-trifluormethyl-1(2H)-pyrimidin, 1-(4-Cyano-2-fluor-5-methoxycarbonylmethylthio-phenyl)-3,6-dihydro-2,6-dioxo-3-methyl-4-trifluormethyl-1(2H)-pyrimidin und 1-[4-Cyano-2-fluor-5-(1-ethoxycarbonyl-ethylthio)-phenyl]-3,6-dihydro-2,6-dioxo-3-methyl-4-trifluormethyl-1(2H)-pyrimidin (vgl. EP-408 382, S. 85 bzw. US-5 084 084, Col. 76) durch Disclaimer ausgenommen sind.

Man erhält die neuen substituierten Cyanophenyluracile der allgemeinen Formel (I), wenn man

(a) substituierte Halogenphenyluracile der allgemeinen Formel (II)

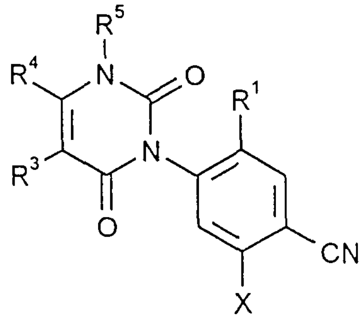

(II)

in welcher

$R^1$, $R^3$, $R^4$ und $R^5$ die oben angegebenen Bedeutungen haben und

X für Halogen steht,

mit nucleophilen Verbindungen der allgemeinen Formel (III)

$$M\text{-}Q\text{-}R^2 \quad \text{(III)}$$

in welcher

Q und $R^2$ die oben angegebenen Bedeutungen haben und

M für Wasserstoff oder ein Alkalimetall steht,

gegebenenfalls in Gegenwart eines Reaktionshilfsmittels und gegebenenfalls in Gegenwart eines Verdünnungsmittels umsetzt,
oder wenn man

(b) substituierte Cyanophenyluracile der allgemeinen Formel (Ia)

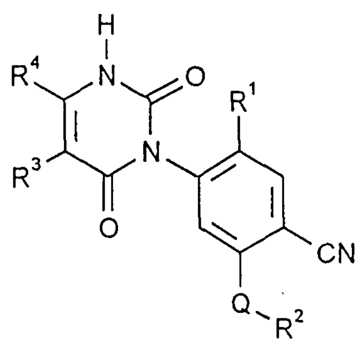

(Ia)

in welcher
Q, $R^1$, $R^2$, $R^3$ und $R^4$ die oben angegebenen Bedeutungen haben,
mit einem Alkylierungsmittel gegebenenfalls in Gegenwart eines Reaktionshilfsmittels und gegebenenfalls in Gegenwart eines Verdünnungsmittels umsetzt.

Die neuen substituierten Cyanophenyluracile der allgemeinen Formel (I) zeichnen sich durch starke herbizide und insektizide Wirksamkeit aus.

In den Definitionen sind die gesättigten oder ungesättigten Kohlenwasserstoffketten, wie Alkyl, Alkenyl oder Alkinyl, jeweils geradkettig oder verzweigt.

Halogen steht im allgemeinen für Fluor, Chlor, Brom oder Iod, vorzugsweise für Fluor, Chlor oder Brom, insbesondere für Fluor oder Chlor.
Gegenstand der Erfindung sind vorzugsweise Verbindungen der Formel (I),
in welcher

Q für O, S, SO oder $SO_2$ steht,

$R^1$ für Wasserstoff, Cyano, Fluor oder Chlor steht,

$R^2$ für Wasserstoff, für jeweils gegebenenfalls durch Cyano, Carboxy, Fluor, Chlor, Brom, $C_1$-$C_4$-Alkoxy, $C_1$-$C_4$-Alkyl-carbonyl, $C_1$-$C_4$-Alkoxy-carbonyl, Di-($C_1$-$C_4$-alkyl)-amino-carbonyl oder N-$C_1$-$C_4$-Alkyl-N-phenyl-aminocarbonyl (wobei die Phenylgruppe gegebenenfalls durch Fluor, Chlor, Brom, Cyano, Nitro, Methyl oder Methoxy substituiert ist) substituiertes Alkyl, Alkenyl oder Alkinyl mit jeweils bis zu 10 Kohlenstoffatomen, für jeweils gegebenenfalls durch Cyano, Carboxy, Fluor, Chlor, Brom, $C_1$-$C_4$-Alkyl, $C_1$-$C_4$-Alkoxy, $C_1$-$C_4$-Alkyl-carbonyl oder $C_1$-$C_4$-Alkoxy-carbonyl substituiertes Cycloalkyl oder Cycloalkylalkyl mit 3 bis 8 Kohlenstoffatomen im Cycloalkylteil und gegebenenfalls bis zu 4 Kohlenstoffatomen im Alkylteil, für jeweils gegebenenfalls durch Cyano, Carboxy, Nitro, Carbamoyl, Thiocarbamoyl, Fluor, Chlor, Brom, durch $C_1$-$C_4$-Alkyl, $C_1$-$C_4$-Alkoxy, $C_1$-$C_4$-Alkylthio, $C_1$-$C_4$-Alkylsulfinyl, $C_1$-$C_4$-Alkylsulfonyl, $C_1$-$C_4$-Alkyl-carbonyl oder $C_1$-$C_4$-Alkoxy-carbonyl (welches jeweils gegebenenfalls durch Fluor und/oder Chlor sind), durch Phenyl, Phenoxy oder Phenylthio (welche jeweils gegebenenfalls durch Fluor, Chlor, Brom, Cyano, Methyl, Methoxy, Trifluormethyl und/oder Trifluormethoxy substituiert sind) substituiertes Aryl oder Arylalkyl mit 6 oder 10 Kohlenstoffatomen im Arylteil und gegebenenfalls bis zu 4 Kohlenstoffatomen im Alkylteil steht,

$R^2$ weiterhin für jeweils gegebenenfalls durch Cyano, Carboxy, Carbamoyl, Thiocarbamoyl, Fluor, Chlor, Brom, durch $C_1$-$C_4$-Alkyl, $C_1$-$C_4$-Alkoxy, $C_1$-$C_4$-Alkylthio, $C_1$-$C_4$-Alkylsulfinyl, $C_1$-$C_4$-Alkylsulfonyl, $C_1$-$C_4$-Alkylcarbonyl oder $C_1$-$C_4$-Alkoxy-carbonyl (welches jeweils gegebenenfalls durch Fluor und/oder Chlor sind), durch Phenyl, Phenoxy oder Phenylthio (welche jeweils gegebenenfalls durch Fluor, Chlor, Brom, Cyano, Methyl, Methoxy, Trifluormethyl und/oder Trifluormethoxy substituiert sind) substituiertes substituiertes Furyl, Tetrahydrofuryl, Thienyl, Tetrahydrothienyl, Oxetanyl, Thietanyl, Oxazolyl, Isoxazolyl, Thiazolyl, Oxadiazolyl, Thiadiazolyl, Pyrazolyl, Pyridinyl, Pyrimidinyl, Triazinyl, Indolyl, Chinolinyl oder Chinoxalinyl steht,

$R^3$ für Wasserstoff, Fluor, Chlor, Brom oder für jeweils gegebenenfalls durch Fluor und/oder Chlor substituiertes Alkyl oder Alkoxy mit jeweils 1 bis 4 Kohlenstoffatomen steht,

$R^4$ für gegebenenfalls durch Fluor und/oder Chlor substituiertes Alkyl mit 1 bis 4 Kohlenstoffatomen steht und

$R^5$ für Wasserstoff oder für jeweils gegebenenfalls durch Fluor, Chlor oder $C_1$-$C_4$-Alkoxy substituiertes Alkyl, Alkoxy, Alkenyl oder Alkinyl mit jeweils bis zu 6 Kohlenstoffatomen steht,

wobei die bekannten Verbindungen 1-(4-Cyano-2-fluor-5-methylthio-phenyl)-3,6-dihydro-2,6-dioxo-3-methyl-4-trifluormethyl-1(2H)-pyrimidin, 1-(4-Cyano-2-fluor-5-methoxycarbonylmethylthio-phenyl)-3,6-dihydro-2,6-dioxo-3-methyl-4-trifluormethyl-1(2H)-pyrimidin und 1-[4-Cyano-2-fluor-5-(1-ethoxycarbonyl-ethylthio)-phenyl]-3,6-dihydro-2,6-dioxo-3-methyl-4-trifluormethyl-1(2H)-pyrimidin (vgl. EP-408 382, S. 85 bzw. US-5 084 084, Col. 76) durch Disclaimer ausgenommen sind.
Die Erfindung betrifft insbesondere Verbindungen der Formel (I),
in welcher

Q für O, S, SO oder $SO_2$ steht,

$R^1$ für Wasserstoff, Fluor oder Chlor steht,

$R^2$ für Wasserstoff, für jeweils gegebenenfalls durch Cyano, Carboxy, Fluor, Chlor, Methoxy, Ethoxy, n- oder i-Propoxy, Acetyl, Propionyl, Methoxycarbonyl, Ethoxycarbonyl, n- oder i-Propoxycarbonyl, Dimethylaminocarbonyl oder Diethylaminocarbonyl substituiertes Methyl, Ethyl, n- oder i-Propyl, n-, i-, s- oder t-Butyl, n-, i-, s- oder t-Pentyl, Propenyl, Butenyl, Pentenyl, Propinyl, Butinyl oder Pentinyl steht,

$R^2$ weiterhin für jeweils gegebenenfalls durch Cyano, Carboxy, Fluor, Chlor, Brom, Methyl, Ethyl, n- oder i-Propyl, Methoxy, Ethoxy, n- oder i-Propoxy, Acetyl oder Propionyl, Methoxycarbonyl, Ethoxycarbonyl, n- oder i-Propoxycarbonyl substituiertes Cyclopropyl, Cyclobutyl, Cyclopentyl, Cyclohexyl, Cyclopropylmethyl, Cyclobutylmethyl, Cyclopentylmethyl oder Cyclohexylmethyl steht,

$R^2$ weiterhin für jeweils gegebenenfalls durch Cyano, Carboxy, Nitro, Carbamoyl, Thiocarbamoyl, Fluor, Chlor, Brom, Methyl, Ethyl, n- oder i-Propyl, n-, i-, s- oder t-Butyl, Trifluormethyl, Methoxy, Ethoxy, n- oder i-Propoxy, Methylthio, Ethylthio, Methylsulfinyl, Ethylsulfinyl, Methylsulfonyl, Ethylsulfonyl, Acetyl, Propionyl, Methoxycarbonyl, Ethoxycarbonyl, n- oder i-Propoxycarbonyl substituiertes Phenyl, Benzyl oder Phenylethyl steht, oder

$R^2$ weiterhin für jeweils gegebenenfalls durch Cyano, Carboxy, Carbamoyl, Thiocarbamoyl, Fluor, Chlor, Brom, Methyl, Ethyl, n- oder i-Propyl, Methoxy, Ethoxy, n- oder i-Propoxy, Methylthio, Ethylthio, Methylsulfinyl, Ethylsulfinyl, Methylsulfonyl, Ethylsulfonyl, Acetyl, Propionyl, Methoxycarbonyl, Ethoxycarbonyl, n- oder i-Propoxycarbonyl, durch Phenyl, Phenoxy oder Phenylthio substituiertes substituiertes Furyl, Tetrahydrofuryl, Thienyl, Tetrahydrothienyl, Oxetanyl, Thietanyl, Oxazolyl, Isoxazolyl, Thiazolyl, Oxadiazolyl, Thiadiazolyl, Pyrazolyl, Pyridinyl, Pyrimidinyl, Triazinyl, Indolyl, Chinolinyl oder Chinoxalinyl steht,

$R^3$ für Wasserstoff, Fluor, Chlor, Brom oder Methyl steht,

$R^4$ für Methyl, Ethyl, Difluormethyl, Dichlormethyl, Trifluormethyl, Trichlormethyl, Chlordifluormethyl, Fluordichlormethyl, Chlorethyl, Fluorethyl, Dichlorethyl, Dichlorethyl, Chlorfluorethyl, Chlordifluorethyl, Fluordichlorethyl, Trifluorethyl, Tetrafluorethyl, Chlortrifluorethyl oder Pentafluorethyl steht und

$R^5$ für Wasserstoff oder für jeweils gegebenenfalls durch Fluor, Chlor, Methoxy oder Ethoxy substituiertes Methyl, Ethyl, n- oder i-Propyl, n-, i-, s-oder t-Butyl, Methoxy, Ethoxy, n- oder i-Propoxy, n-, i- oder s-Butoxy, Propenyl, Butenyl, Propinyl oder Butinyl steht,

wobei die bekannten Verbindungen 1-(4-Cyano-2-fluor-5-methylthio-phenyl)-3,6-dihydro-2,6-dioxo-3-methyl-4-trifluormethyl-1(2H)-pyrimidin, 1-(4-Cyano-2-fluor-5-methoxycarbonylmethylthio-phenyl)-3,6-dihydro-2,6-dioxo-3-methyl-4-trifluormethyl-1(2H)-pyrimidin und 1-[4-Cyano-2-fluor-5-(1-ethoxycarbonyl-ethylthio)-phenyl]-3,6-dihydro-2,6-dioxo-3-methyl-4-trifluormethyl-1(2H)-pyrimidin (vgl. EP-408 382, S. 85 bzw. US-5 084 084, Col. 76) durch Disclaimer ausgenommen sind.

Die oben aufgeführten allgemeinen oder in Vorzugsbereichen aufgeführten Restedefinitionen gelten sowohl für die Endprodukte der Formel (I) als auch entsprechend für die jeweils zur Herstellung benötigten Ausgangsstoffe bzw. Zwischenprodukte. Diese Restedefinitionen können untereinander, also auch zwischen den angegebenen bevorzugten Bereichen beliebig kombiniert werden.

Beispiele für die erfindungsgemäßen Verbindungen der Formel (I) sind in den nachstehenden Gruppen aufgeführt.

## Gruppe 1

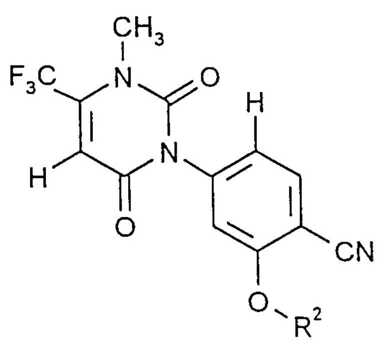

$R^2$ hat hierbei beispielhaft die in der nachstehenden Aufzählung angegebenen Bedeutungen:

Wasserstoff, Methyl, Ethyl, n- oder i-Propyl, n-, i-, s- oder t-Butyl, n-, i-, s- oder t-Pentyl, Difluormethyl, Trifluormethyl, Chlordifluormethyl, Fluordichlormethyl, Fluorethyl, Chlorethyl, Chlorfluorethyl, Difluorethyl, Dichlorethyl, Trifluorethyl, Trichlorethyl, Chlordifluorethyl, Tetrafluorethyl, Chlortrifluorethyl, Pentafluorethyl, Fluorpropyl, Chlorpropyl, Difluorpropyl, Dichlorpropyl, Trifluorpropyl, Trifluorpropyl, Cyanomethyl, Cyanoethyl, Cyanopropyl, Cyanobutyl,

Carboxymethyl, Carboxyethyl, Carboxypropyl, Carboxybutyl, Methoxymethyl, Ethoxymethyl, Propoxymethyl, Methoxyethyl, Ethoxyethyl, Propoxyethyl, Methoxypropyl, Ethoxypropyl, Propoxypropyl, Methoxycarbonylmethyl, Ethoxycarbonylmethyl, Propoxycarbonylmethyl, Methoxycarbonylethyl, Ethoxycarbonylethyl, Propoxycarbonylethyl, Methoxycarbonylpropyl, Ethoxycarbonylpropyl, Propoxycarbonylpropyl, 1-Propen-3-yl (Allyl), 3-Methyl-1-propen-3-yl, 2-Buten-4-yl (Crotonyl), 1-Propin-3-yl (Propargyl), 3-Methyl-1-propin-3-yl, 2-Butin-4-yl, Cyclopropyl, Cyanocyclopropyl, Carboxycyclopropyl, Difluorcyclopropyl, Dichlorcyclopropyl, Methylcyclopropyl, Methoxycarbonylcyclopropyl, Ethoxycarbonylcyclopropyl, Cyclobutyl, Cyanocyclobutyl, Carboxycyclobutyl, Difluorcyclopropyl, Trifluorcyclobutyl, Tetrafluorcyclobutyl, Chlortrifluorcyclobutyl, Methylcyclobutyl, Cyclopentyl, Cyanocyclopentyl, Carboxycyclopentyl, Fluorcyclopentyl, Chlorcyclopentyl, Difluorcyclopentyl, Dichlorcyclopentyl, Methylcyclopentyl, Methoxycarbonylcyclopentyl, Ethoxycarbonylcyclopentyl, Cyclohexyl, Cyanocyclohexyl, Carboxycyclohexyl, Fluorcyclohexyl, Chlorcyclohexyl, Difluorcyclohexyl, Dichlorcyclohexyl, Methylcyclohexyl, Trifluormethylcyclohexyl, Methoxycarbonylcyclohexyl, Ethoxycarbonylcyclohexyl, Cyclopropylmethyl, Difluorcyclopropylmethyl, Dichlorcyclopropylmethyl, Cyclobutylmethyl, Cyclopentylmethyl, Cyclohexylmethyl, Cyanocyclohexylmethyl, Carboxycyclohexylmethyl, Fluorcyclohexylmethyl, Chlorcyclo-hexylmethyl, Methylcyclohexylmethyl, Trifluormethylcyclohexylmethyl, Phenyl, Cyanophenyl, Carboxyphenyl, Nitrophenyl, Fluorphenyl, Chlorphenyl, Bromphenyl, Methylphenyl, Trifluormethylphenyl, Methoxyphenyl, Difluormethoxyphenyl, Trifluormethoxyphenyl, Methoxycarbonylphenyl, Ethoxycarbonylphenyl, Benzyl, Cyanobenzyl, Carboxybenzyl, Fluorbenzyl, Chlorbenzyl, Methylbenzyl, Trifluormethylbenzyl, Methoxybenzyl, Difluormethoxybenzyl, Trifluormethoxybenzyl, Methoxycarbonylbenzyl, Ethoxycarbonylbenzyl, Phenylethyl, Furyl, Tetrahydrofuryl, Thienyl, Tetrahydrothienyl, Oxetanyl, Oxazolyl, Isoxazolyl.

## Gruppe 2

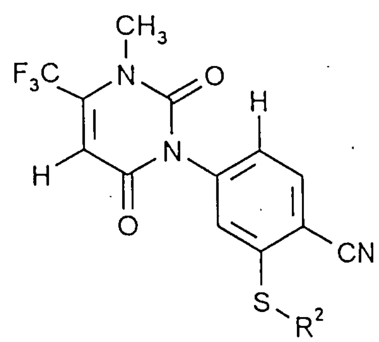

$R^2$ hat hierbei beispielhaft die oben in Gruppe 1 aufgeführten Bedeutungen.

## Gruppe 3

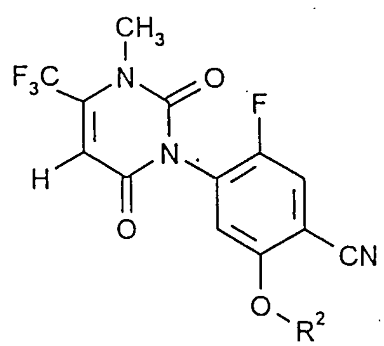

$R^2$ hat hierbei beispielhaft die oben in Gruppe 1 aufgeführten Bedeutungen.

## Gruppe 4

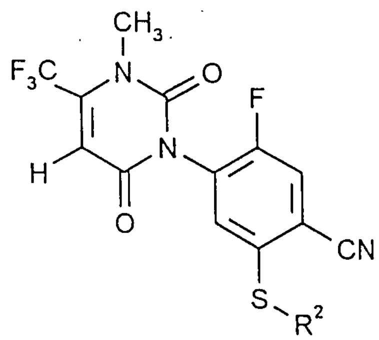

$R^2$ hat hierbei beispielhaft die oben in Gruppe 1 aufgeführten Bedeutungen.

## Gruppe 5

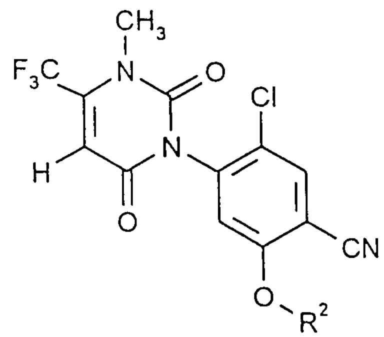

$R^2$ hat hierbei beispielhaft die oben in Gruppe 1 aufgeführten Bedeutungen.

## Gruppe 6

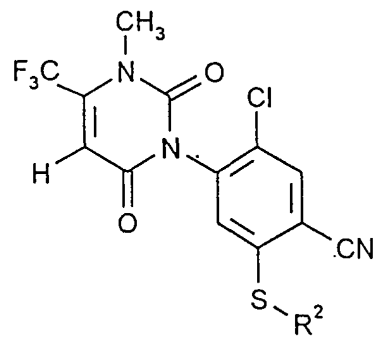

$R^2$ hat hierbei beispielhaft die oben in Gruppe 1 aufgeführten Bedeutungen.

## Gruppe 7

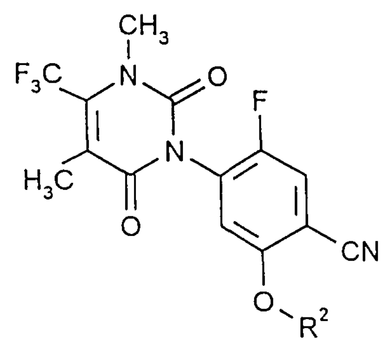

$R^2$ hat hierbei beispielhaft die oben in Gruppe 1 aufgeführten Bedeutungen.

## Gruppe 8

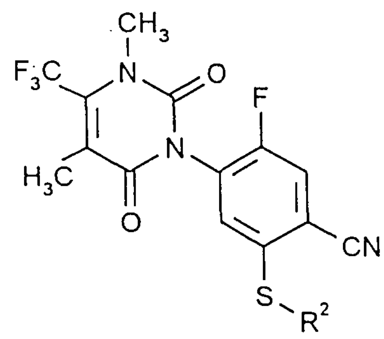

$R^2$ hat hierbei beispielhaft die oben in Gruppe 1 aufgeführten Bedeutungen.

## Gruppe 9

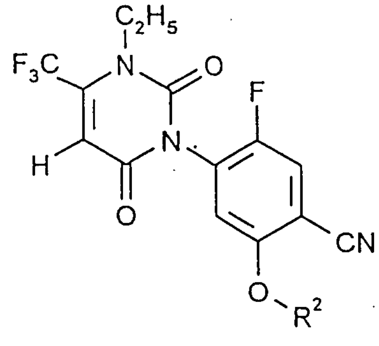

$R^2$ hat hierbei beispielhaft die oben in Gruppe 1 aufgeführten Bedeutungen.

## Gruppe 10

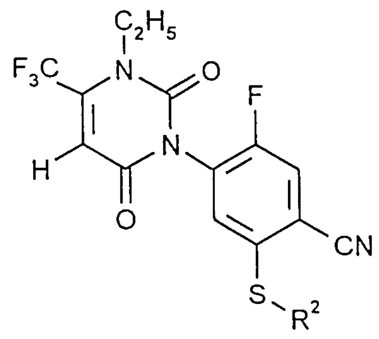

$R^2$ hat hierbei beispielhaft die oben in Gruppe 1 aufgeführten Bedeutungen.

## Gruppe 11

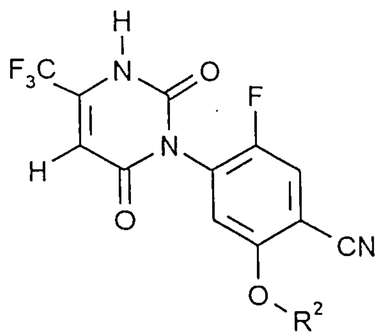

$R^2$ hat hierbei beispielhaft die oben in Gruppe 1 aufgeführten Bedeutungen.

## Gruppe 12

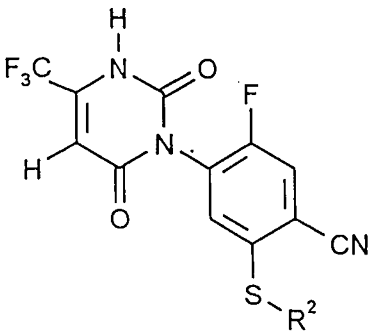

$R^2$ hat hierbei beispielhaft die oben in Gruppe 1 aufgeführten Bedeutungen.

## Gruppe 13

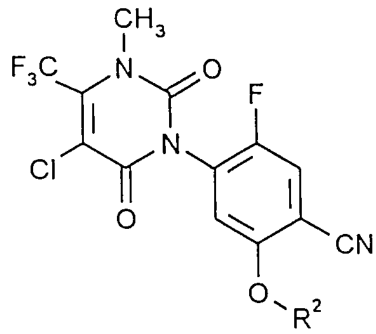

$R^2$ hat hierbei beispielhaft die oben in Gruppe 1 aufgeführten Bedeutungen.

## Gruppe 14

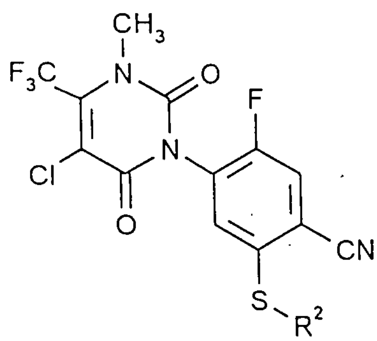

$R^2$ hat hierbei beispielhaft die oben in Gruppe 1 aufgeführten Bedeutungen.

## Gruppe 15

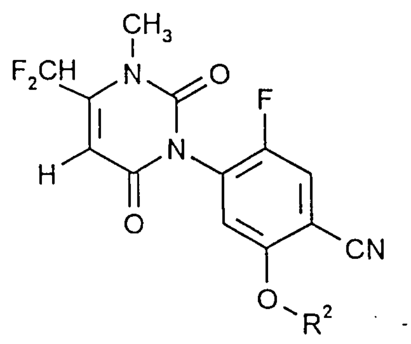

$R^2$ hat hierbei beispielhaft die oben in Gruppe 1 aufgeführten Bedeutungen.

## Gruppe 16

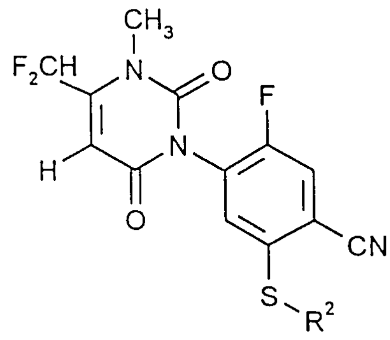

$R^2$ hat hierbei beispielhaft die oben in Gruppe 1 aufgeführten Bedeutungen.

## Gruppe 17

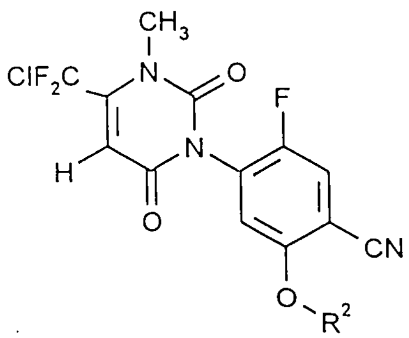

$R^2$ hat hierbei beispielhaft die oben in Gruppe 1 aufgeführten Bedeutungen.

## Gruppe 18

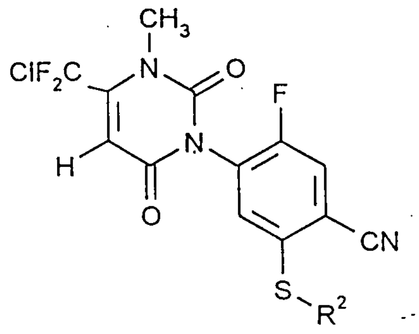

$R^2$ hat hierbei beispielhaft die oben in Gruppe 1 aufgeführten Bedeutungen.

**Gruppe 19**

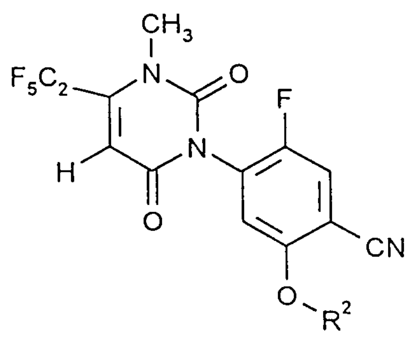

$R^2$ hat hierbei beispielhaft die oben in Gruppe 1 aufgeführten Bedeutungen.

**Gruppe 20**

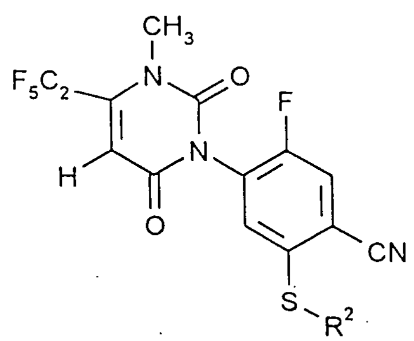

$R^2$ hat hierbei beispielhaft die oben in Gruppe 1 aufgeführten Bedeutungen.

Verwendet man beispielsweise 1-(4-Cyano-2,5-difluor-phenyl)-3,6-dihydro-2,6-dioxo-5-chlor-4-difluormethyl-3-methyl-1(2H)-pyrimidin und Kaliumethylat als Ausgangsstoffe, so kann der Reaktionsablauf beim erfindungsgemäßen Verfahren (a) durch das folgende Formelschema skizziert werden:

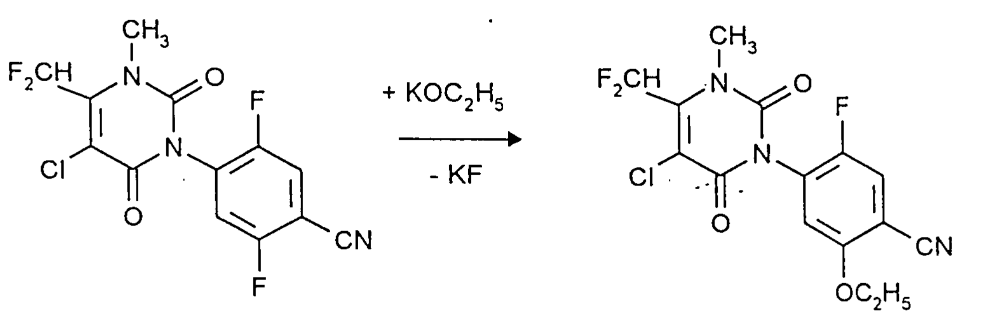


Verwendet man beispielsweise 1-(2-Chlor-4-cyano-5-methylthio-phenyl)-3,6-dihydro-2,6-dioxo-4-chlordifluormethyl-5-methyl-1(2H)-pyrimidin und Brommethan als Ausgangsstoffe, so kann der Reaktionsablauf beim erfindungsgemäßen Verfahren (b) durch das folgende Formelschema skizziert werden:

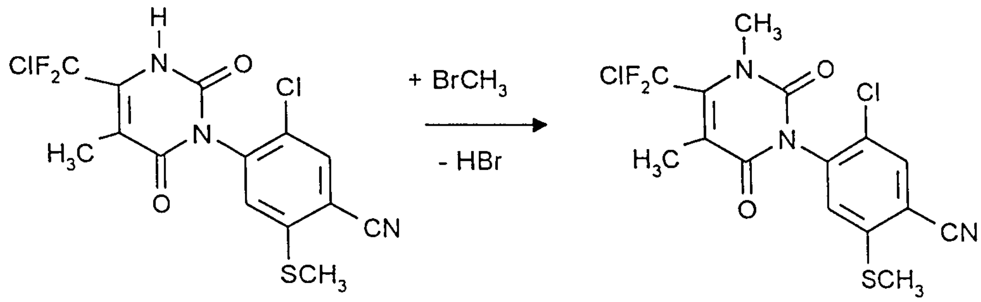


Die beim erfindungsgemäßen Verfahren zur Herstellung der Verbindungen der Formel (I) als Ausgangsstoffe zu verwendenden substituierten Halogenphenyluracile sind durch die Formel (II) allgemein definiert. In der Formel (II) haben $R^1$, $R^3$, $R^4$ und $R^5$ vorzugsweise bzw. insbesondere diejenige Bedeutung, die bereits oben bei der Beschreibung der erfindungsgemäß herzustellenden Verbindungen der Formel (I) vorzugsweise bzw. als insbesondere bevorzugt für $R^1$, $R^3$, $R^4$ und $R^5$ angegeben wurde; X steht vorzugsweise für Fluor, Chlor oder Brom, insbesondere für Fluor.

Die Ausgangsstoffe der Formel (II) sind bekannt und/oder können nach an sich bekannten Verfahren hergestellt werden (vgl. EP 648749, Herstellungsbeispiele).

Die beim erfindungsgemäßen Verfahren zur Herstellung der Verbindungen der Formel (I) weiter als Ausgangsstoffe zu verwendenden nucleophilen Verbindungen sind durch die Formel (III) allgemein definiert. In der Formel (III) haben Q und $R^2$ vorzugsweise bzw. insbesondere diejenige Bedeutung, die bereits oben bei der Beschreibung der erfindungsgemäß herzustellenden Verbindungen der Formel (I) vorzugsweise bzw. als insbesondere bevorzugt für Q und $R^2$ angegeben wurden; M steht vorzugsweise für Wasserstoff, Lithium, Natrium oder Kalium, insbesondere für Wasserstoff, Natrium oder Kalium.

Die Ausgangsstoffe der Formel (III) sind bekannte Synthesechemikalien.

Das erfindungsgemäße Verfahren (a) zur Herstellung der Verbindungen der Formel (I) wird vorzugsweise in Gegenwart eines geeigneten Reaktionshilfsmittels durchgeführt. Als Reaktionshilfsmittel kommen im allgemeinen die üblichen anorganischen oder organischen Basen oder Säureakzeptoren in Betracht. Hierzu gehören vorzugsweise Alkalimetall- oder Erdalkalimetall- -acetate, -amide, -carbonate, -hydrogencarbonate, -hydride, -hydroxide oder -alkanolate, wie beispielsweise Natrium-, Kalium- oder Calcium-acetat, Lithium-, Natrium-, Kalium- oder Calciumamid, Natrium-, Kalium- oder Calcium-carbonat, Natrium-, Kalium- oder Calcium-hydrogencarbonat, Lithium-, Natrium-, Kalium- oder Calcium-hydrid, Lithium-, Natrium-, Kalium- oder Calcium-hydroxid, Natrium- oder Kalium- -methanolat, -ethanolat, n- oder i-propanolat, n-, i-, s- oder t-butanolat; weiterhin auch basische organische Stickstoffverbindungen, wie beispielsweise Trimethylamin, Triethylamin, Tripropylamin, Tributylamin, Ethyl-diisopropylamin, N,N-Dimethyl-cyclohexylamin, Dicyclohexylamin, Ethyl-dicyclohexylamin, N,N-Dimethyl-anilin, N,N-Dimethyl-benzylamin, Pyridin, 2-Methyl-, 3-Methyl-, 4-Methyl-, 2,4-Dimethyl-, 2,6-Dimethyl-, 3,4-Dimethyl- und 3,5-Dimethyl-pyridin, 5-Ethyl-2-methyl-pyridin, 4-Dimethylamino-pyridin, N-Methyl-piperidin, 1,4-Diazabicyclo[2,2,2]-octan (DABCO), 1,5-Diazabicyclo[4,3,0]-non-5-en (DBN), und 1,8 Diazabicyclo[5,4,0]-undec-7-en (DBU).

Das erfindungsgemäße Verfahren (a) zur Herstellung der Verbindungen der Formel (I) wird vorzugsweise in Gegenwart eines Verdünnungsmittels durchgeführt. Als Verdünnungsmittel kommen im allgemeinen die üblichen organischen Lösungsmittel in Betracht. Hierzu gehören vorzugsweise aliphatische, alicyclische und aromatische, gegebenenfalls halogenierte Kohlenwasserstoffe, wie beispielsweise Pentan, Hexan, Heptan, Petrolether, Ligroin, Benzin, Benzol, Toluol, Xylol, Chlorbenzol, Dichlorbenzol, Cyclohexan, Methylcyclohexan, Dichlormethan (Methylenchlorid), Trichlormethan (Chloroform) oder Tetrachlormethan, Dialkylether, wie beispielsweise Diethylether, Diisopropylether, Methyl-t-butylether (MTBE), Ethylt-butylether, Methyl-t-pentylether (TAME), Ethyl-t-pentylether, Tetrahydrofuran (THF), 1,4-Dioxan, Ethylenglycol-dimethylether oder -diethylether, Diethylenglycol-dimethylether oder -diethylether; Dialkylketone, wie beispielsweise Aceton, Butanon (Methylethylketon), Methyl-i-propylketon oder Methyl-i-butylketon, Nitrile, wie beispielsweise Acetonitril, Propionitril, Butyronitril oder Benzonitril; Amide, wie beispielsweise N,N-Dimethylformamid (DMF), N,N-Dimethyl-acetamid, N-Methyl-formanilid, N-Methyl-pyrrolidon oder Hexamethyl-phosphorsäuretriamid; Ester, wie beispielsweise Essigsäure-methylester, -ethylester, -n- oder -ipropylester, -n-, -i- oder -s-butylester; Sulfoxide, wie beispielsweise Dimethylsulfoxid; Alkanole, wie beispielsweise Methanol, Ethanol, n- oder i-Propanol, n-, i-, s- oder t-Butanol, Ethylenglycol-monomethylether oder -monoethylether, Diethylenglycol-monomethylether oder -monoethylether; deren Gemische mit Wasser oder reines Wasser.

Die Reaktionstemperaturen können bei der Durchführung des erfindungsgemäßen Verfahrens (a) in einem größeren Bereich variiert werden. Im allgemeinen arbeitet man bei Temperaturen zwischen 0°C und 200°C, vorzugsweise zwischen 10°C und 150°C.

Das erfindungsgemäße Verfahren (a) wird im allgemeinen unter Normaldruck durchgeführt. Es ist jedoch auch möglich, das erfindungsgemäße Verfahren unter erhöhtem oder vermindertem Druck - im allgemeinen zwischen 0,1 bar und 10 bar - durchzuführen.

Zur Durchführung des erfindungsgemäßen Verfahrens (a) werden die Ausgangsstoffe im allgemeinen in angenähert äquimolaren Mengen eingesetzt. Es ist jedoch auch möglich, eine der Komponenten in einem größeren Überschuß zu verwenden. Die Umsetzung wird im allgemeinen in einem geeigneten Verdünnungsmittel in Gegenwart eines Reaktionshilfsmittels durchgeführt und das Reaktionsgemisch wird im allgemeinen mehrere Stunden bei der erforderlichen Temperatur gerührt. Die Aufarbeitung wird nach üblichen Methoden durchgeführt (vgl. die Herstellungsbeispiele).

Die beim erfindungsgemäßen Verfahren (b) zur Herstellung der Verbindungen der Formel (I) als Ausgangsstoffe zu verwendenden substituierten Cyanophenyluracile sind durch die Formel (Ia) allgemein definiert. In der Formel (Ia) haben Q, $R^1$, $R^2$, $R^3$ und $R^4$ vorzugsweise bzw. insbesondere diejenige Bedeutung, die bereits oben bei der Beschreibung der erfindungsgemäß herzustellenden Verbindungen der Formel (I) vorzugsweise bzw. als insbesondere bevorzugt für Q, $R^1$, $R^2$, $R^3$ und $R^4$ angegeben wurde.

Die Ausgangsstoffe der Formel (Ia) sind erfindungsgemäße, neue Verbindungen; sie können nach dem erfindungsgemäßen Verfahren (a) hergestellt werden.

Das erfindungsgemäße Verfahren (b) wird unter Verwendung eines Alkylierungsmittels durchgeführt. Unter Alkylierungsmitteln sind in diesem Zusammenhang vorzugsweise gegebenenfalls durch Fluor, Chlor oder $C_1$-$C_4$-Alkoxy substituierte Alkyl-, Alkenyl- oder Alkinylhalogenide (insbesondere Chloride, Bromide oder Iodide) mit jeweils bis zu 6 Kohlenstoffatomen oder Dialkylsulfate mit bis zu 6 Kohlenstoffatomen in den Alkylgruppen zu verstehen. Es handelt sich hierbei um bekannte Synthesechemikalien.

Das erfindungsgemäße Verfahren (b) zur Herstellung der Verbindungen der Formel (I) wird vorzugsweise in Gegenwart eines geeigneten Reaktionshilfsmittels durchgeführt. Als Reaktionshilfsmittel kommen im allgemeinen die üblichen anorganischen oder organischen Basen oder Säureakzeptoren in Betracht. Hierzu gehören vorzugsweise Alkalimetall- oder Erdalkalimetall- -acetate, -amide, -carbonate, -hydrogencarbonate, -hydride, -hydroxide oder -alkanolate, wie beispielsweise Natrium-, Kalium- oder Calcium-acetat, Lithium-, Natrium-, Kalium- oder Calcium-amid, Natrium-, Kalium- oder Calcium-carbonat, Natrium-, Kalium- oder Calcium-hydrogencarbonat, Lithium-, Natrium-, Kalium- oder Calcium-hydrid, Lithium-, Natrium-, Kalium- oder Calcium-hydroxid, Natrium- oder Kaliummethanolat, -ethanolat, n- oder i-propanolat, n-, i-, s- oder t-butanolat; weiterhin auch basische organische Stickstoffverbindungen, wie beispielsweise Trimethylamin, Triethylamin, Tripropylamin, Tributylamin, Ethyl-diisopropylamin, N,N-Dimethyl-cyclohexylamin, Dicyclohexylamin, Ethyl-dicyclohexylamin, N,N-Dimethyl-anilin, N,N-Dimethyl-benzylamin, Pyridin, 2-Methyl-, 3-Methyl-, 4-Methyl-, 2,4-Dimethyl-, 2,6-Dimethyl-, 3,4-Dimethyl- und 3,5-Dimethyl-pyridin, 5-Ethyl-2-methyl-pyridin, 4-Dimethylamino-pyridin, N-Methyl-piperidin, 1,4-Diazabicyclo-[2,2,2]-octan (DABCO), 1,5-Diazabicyclo[4,3,0]-non-5-en (DBN), und 1,8 Diazabicyclo[5,4,0]-undec-7-en (DBU).

Das erfindungsgemäße Verfahren (b) zur Herstellung der Verbindungen der Formel (I) wird vorzugsweise in Gegenwart eines Verdünnungsmittels durchgeführt. Als Verdünnungsmittel kommen im allgemeinen die üblichen organischen Lösungsmittel in Betracht. Hierzu gehören vorzugsweise aliphatische, alicyclische und aromatische, gegebenenfalls halogenierte Kohlenwasserstoffe, wie beispielsweise Pentan, Hexan, Heptan, Petrolether, Ligroin, Benzin, Benzol, Toluol, Xylol, Chlorbenzol, Dichlorbenzol, Cyclohexan, Methylcyclohexan, Dichlormethan (Methylenchlorid), Trichlormethan (Chloroform) oder Tetrachlormethan, Dialkylether, wie beispielsweise Diethylether, Diisopropylether, Methyl-t-butylether (MTBE), Ethylt-butylether, Methyl-t-pentylether (TAME), Ethyl-t-pentylether, Tetrahydrofuran (THF), 1,4-Dioxan, Ethylenglycol-dimethylether oder -diethylether, Diethylenglycol-dimethylether oder -diethylether; Dialkylketone, wie beispielsweise Aceton, Butanon (Methylethylketon), Methyl-i-propylketon oder Methyl-i-butylketon, Nitrile, wie beispielsweise Acetonitril, Propionitril, Butyronitril oder Benzonitril; Amide, wie beispielsweise N,N-Dimethylformamid (DMF), N,N-Dimethyl-acetamid, N-Methyl-formanilid, N-Methyl-pyrrolidon oder Hexamethyl-phosphorsäuretriamid; Ester, wie beispielsweise Essigsäure-methylester, -ethylester, -n- oder -ipropylester, -n-, -i- oder -s-butylester; Sulfoxide, wie beispielsweise Dimethylsulfoxid; Alkanole, wie beispielsweise Methanol, Ethanol, n- oder i-Propanol, n-, i-, s- oder t-Butanol, Ethylenglycol-monomethylether oder -monoethylether, Diethylenglycol-monomethylether oder -monoethylether; deren Gemische mit Wasser oder reines Wasser.

Die Reaktionstemperaturen können bei der Durchführung des erfindungsgemäßen Verfahrens (b) in einem größeren Bereich variiert werden. Im allgemeinene arbeitet man bei Temperaturen zwischen 0°C und 150°C, vorzugsweise zwischen 10°C und 120°C.

Das erfindungsgemäße Verfahren (b) wird im allgemeinen unter Normaldruck durchgeführt. Es ist jedoch auch möglich, das erfindungsgemäße Verfahren unter erhöhtem oder vermindertem Druck - im allgemeinen zwischen 0,1 bar und 10 bar - durchzuführen.

Zur Durchführung des erfindungsgemäßen Verfahrens werden die Ausgangsstoffe im allgemeinen in angenähert äquimolaren Mengen eingesetzt. Es ist jedoch auch möglich, eine der Komponenten in einem größeren Überschuß zu verwenden. Die Umsetzung wird im allgemeinen in einem geeigneten Verdünnungsmittel in Gegenwart eines

Reaktionshilfsmittels durchgeführt und das Reaktionsgemisch wird im allgemeinen mehrere Stunden bei der erforderlichen Temperatur gerührt. Die Aufarbeitung wird nach üblichen Methoden durchgeführt (vgl. die Herstellungsbeispiele).

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

<u>Dikotyle Unkräuter der Gattungen:</u> Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea, Trifolium, Ranunculus, Taraxacum.

<u>Dikotyle Kulturen der Gattungen:</u> Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

<u>Monokotyle Unkräuter der Gattungen:</u> Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

<u>Monokotyle Kulturen der Gattungen:</u> Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfruchtund Hopfenanlagen, auf Zier- und Sportrasen und Weideflächen und zur selektiven Unkrautbekämpfung in einjährigen Kulturen eingesetzt werden.

Die erfindungsgemäßen Verbindungen der Formel (I) eignen sich insbesondere zur selektiven Bekämpfung von monokotylen und dikotylen Unkräutern in monokotylen und dikotylen Kulturen sowohl im Vorauflauf- als auch im NachauflaufVerfahren.

Weiter zeigen die erfindungsgemäßen Verbindungen der Formel (I) auch starke insektizide Wirksamkeit, vor allem gegen Käferlarven, wie z.B. Phaedon cochleariae, und gegen Schmetterlingsraupen, wie z.B. Plutella xylostella.

In gewissem Umfang zeigen die Verbindungen der Formel (I) auch fungizide Wirkung, beispielsweise gegen Pyricularia oryzae an Reis.

Die Wirkstoffe eignen sich bei guter Pflanzenverträglichkeit und günstiger Warmblütertoxizität zur Bekämpfung von tierischen Schädlingen, insbesondere Insekten, Spinnentieren und Nematoden, die in der Landwirtschaft, in Forsten, im Vorratsund Materialschutz sowie auf dem Hygienesektor vorkommen. Sie können vorzugsweise als Pflanzenschutzmittel eingesetzt werden. Sie sind gegen normal sensible und resistente Arten sowie gegen alle oder einzelne Entwicklungsstadien wirksam. Zu den oben erwähnten Schädlingen gehören:

Aus der Ordnung der Isopoda z.B. Oniscus asellus, Armadillidium vulgare, Porcellio scaber.

Aus der Ordnung der Diplopoda z.B. Blaniulus guttulatus.

Aus der Ordnung der Chilopoda z.B. Geophilus carpophagus, Scutigera spec.

Aus der Ordnung der Symphyla z.B. Scutigerella immaculata.

Aus der Ordnung der Thysanura z.B. Lepisma saccharina.

Aus der Ordnung der Collembola z.B. Onychiurus armatus.

Aus der Ordnung der Orthoptera z.B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.

Aus der Ordnung der Dermaptera z.B. Forficula auricularia.

Aus der Ordnung der Isoptera z.B. Reticulitermes spp..

Aus der Ordnung der Anoplura z.B. Pediculus humanus corporis, Haematopinus spp., Linognathus spp.

Aus der Ordnung der Mallophaga z.B. Trichodectes spp., Damalinea spp.

Aus der Ordnung der Thysanoptera z.B. Hercinothrips femoralis, Thrips tabaci.

Aus der Ordnung der Heteroptera z.B. Eurygaster spp., Dysdercus intermedius, Piesma quadrata, Cimex

lectularius, Rhodnius prolixus, Triatoma spp.

Aus der Ordnung der Homoptera z.B. Aleurodes brassicae, Bemisia tabaci, Trialeurodes vaporariorum, Aphis gossypii, Brevicoryne brassicae, Cryptomyzus ribis, Aphis fabae, Aphis pomi, Eriosoma lanigerum, Hyalopterus arundinis, Phylloxera vastatrix, Pemphigus spp., Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp., Psylla spp.

Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp., Bucculatrix thurberiella, Phyllocnistis citrella, Agrotis spp., Euxoa spp., Feltia spp., Earias insulana, Heliothis spp., Spodoptera exigua, Mamestra brassicae, Panolis flammea, Spodoptera litura, Spodoptera spp., Trichoplusia ni, Carpocapsa pomonella, Pieris spp., Chilo spp., Pyrausta nubilalis, Ephestia kuehniella, Galleria mellonella, Tineola bisselliella, Tinea pellionella, Hofmannophila pseudospretella, Cacoecia podana, Capua reticulana, Choristoneura fumiferana, Clysia ambiguella, Homona magnanima, Tortrix viridana.

Aus der Ordnung der Coleoptera z.B. Anobium punctatum, Rhizopertha dominica, Bruchidius obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica.

Aus der Ordnung der Hymenoptera z.B. Diprion spp., Hoplocampa spp., Lasius spp., Monomorium pharaonis, Vespa spp.

Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.

Aus der Ordnung der Siphonaptera z.B. Xenopsylla cheopis, Ceratophyllus spp..

Aus der Ordnung der Arachnida z.B. Scorpio maurus, Latrodectus mactans.

Aus der Ordnung der Acarina z.B. Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp..

Zu den pflanzenparasitären Nematoden gehören z.B. Pratylenchus spp., Radopholus similis, Ditylenchus dipsaci, Tylenchulus semipenetrans, Heterodera spp., Globodera spp., Meloidogyne spp., Aphelenchoides spp., Longidorus spp., Xiphinema spp., Trichodorus spp..

Zu Anwendung geeignete Mischpartner sind z.B. die folgenden:

**Fungizide:**

2-Aminobutan; 2-Anilino-4-methyl-6-cyclopropyl-pyrimidin; 2',6'-Dibromo-2-methyl-4'-trifluoromethoxy-4'-trifluoromethyl-1,3-thiazol-5-carboxanilid; 2,6-DichloroN-(4-trifluoromethylbenzyl)-benzamid; (E)-2-Methoxyimino-N-methyl-2-(2-phenoxyphenyl)-acetamid; 8-Hydroxyquinolinsulfat; Methyl-(E)-2-{2-[6-(2-cyanophenoxy)-pyrimidin-4-yloxy]-phenyl}-3-methoxyacrylat; Methyl-(E)-methoximino-[alpha-(o-tolyloxy)-o-tolyl]acetat; 2-Phenylphenol (OPP), Aldimorph, Ampropylfos, Anilazin, Azaconazol,

Benalaxyl, Benodanil, Benomyl, Binapacryl, Biphenyl, Bitertanol, Blasticidin-S, Bromuconazole, Bupirimate, Buthiobate,

Calciumpolysulfid, Captafol, Captan, Carbendazim, Carboxin, Chinomethionat (Quinomethionat), Chloroneb, Chloropicrin, Chlorothalonil, Chlozolinat, Cufraneb, Cymoxanil, Cyproconazole, Cyprofuram,

Dichlorophen, Diclobutrazol, Diclofluanid, Diclomezin, Dicloran, Diethofencarb, Difenoconazol, Dimethirimol, Dimethomorph, Diniconazol, Dinocap, Diphenylamin, Dipyrithion, Ditalimfos, Dithianon, Dodine, Drazoxolon,

Edifenphos, Epoxyconazole, Ethirimol, Etridiazol,

Fenarimol, Fenbuconazole, Fenfuram, Fenitropan, Fenpiclonil, Fenpropidin, Fenpropimorph, Fentinacetat, Fentinhydroxyd, Ferbam, Ferimzone, Fluazinam, Fludioxonil, Fluoromide, Fluquinconazole, Flusilazole, Flusulfamide, Flutolanil, Flutriafol, Folpet, Fosetyl-Aluminium, Fthalide, Fuberidazol, Furalaxyl, Furmecyclox,

Guazatine,

Hexachlorobenzol, Hexaconazol, Hymexazol,

Imazalil, Imibenconazol, Iminoctadin, Iprobenfos (IBP), Iprodion, Isoprothiolan,

Kasugamycin, Kupfer-Zubereitungen, wie: Kupferhydroxid, Kupfernaphthenat, Kupferoxychlorid, Kupfersulfat, Kupferoxid, Oxin-Kupfer und Bordeaux-Mischung,

Mancopper, Mancozeb, Maneb, Mepanipyrim, Mepronil, Metalaxyl, Metconazol, Methasulfocarb, Methfuroxam, Metiram, Metsulfovax, Myclobutanil,

Nickel-dimethyldithiocarbamat, Nitrothal-isopropyl, Nuarimol,

Ofurace, Oxadixyl, Oxamocarb, Oxycarboxin,

Pefurazoat, Penconazol, Pencycuron, Phosdiphen, Phthalid, Pimaricin, Piperalin, Polycarbamate, Polyoxin, Probenazol, Prochloraz, Procymidon, Propamocarb, Propiconazole, Propineb, Pyrazophos, Pyrifenox, Pyrimethanil, Pyroquilon,

Quintozen (PCNB),

Schwefel und Schwefel-Zubereitungen,

Tebuconazol, Tecloftalam, Tecnazen, Tetraconazol, Thiabendazol, Thicyofen, Thiophanat-methyl, Thiram, Tolclophos-methyl, Tolylfluanid, Triadimefon, Triadimenol, Triazoxid, Trichlamid, Tricyclazol, Tridemorph, Triflumizol, Triforin, Triticonazol,

Validamycin A, Vinclozolin,

Zineb, Ziram.

**Bakterizide:**

Bronopol, Dichlorophen, Nitrapyrin, Nickel-Dimethyldithiocarbamat, Kasugamycin, Octhilinon, Furancarbonsäure, Oxytetracyclin, Probenazol, Streptomycin, Tecloftalam, Kupfersulfat und andere Kupfer-Zubereitungen.

**Insektizide / Akarizide / Nematizide:**

Abamectin, AC 303 630, Acephat, Acrinathrin, Alanycarb, Aldicarb, Alphamethrin, Amitraz, Avermectin, AZ 60541, Azadirachtin, Azinphos A, Azinphos M, Azocyclotin,

Bacillus thuringiensis, Bendiocarb, Benfuracarb, Bensultap, Betacyfluthrin, Bifenthrin, BPMC, Brofenprox, Bromophos A, Bufencarb, Buprofezin, Butocarboxin, Butylpyridaben,

Cadusafos, Carbaryl, Carbofuran, Carbophenothion, Carbosulfan, Cartap, CGA 157 419, CGA 184699, Chloethocarb, Chlorethoxyfos, Chlorfenvinphos, Chlorfluazuron, Chlormephos, Chlorpyrifos, Chlorpyrifos M, Cis-Resmethrin, Clocythrin, Clofentezin, Cyanophos, Cycloprothrin, Cyfluthrin, Cyhalothrin, Cyhexatin, Cypermethrin, Cyromazin,

Deltamethrin, Demeton M, Demeton S, Demeton-S-methyl, Diafenthiuron, Diazinon, Dichlofenthion, Dichlorvos, Dicliphos, Dicrotophos, Diethion, Diflubenzuron, Dimethoat, Dimethylvinphos, Dioxathion, Disulfoton,

Edifenphos, Emamectin, Esfenvalerat, Ethiofencarb, Ethion, Ethofenprox, Ethoprophos, Etrimphos,

Fenamiphos, Fenazaquin, Fenbutatinoxid, Fenitrothion, Fenobucarb, Fenothiocarb, Fenoxycarb, Fenpropathrin, Fenpyrad, Fenpyroximat, Fenthion, Fenvalerate, Fipronil, Fluazinam, Flucycloxuron, Flucythrinat, Flufenoxuron, Flufenprox, Fluvalinate, Fonophos, Formothion, Fosthiazat, Fubfenprox, Furathiocarb,

HCH, Heptenophos, Hexaflumuron, Hexythiazox,

Imidacloprid, Iprobenfos, Isazophos, Isofenphos, Isoprocarb, Isoxathion, Ivermectin, Lambda-cyhalothrin, Lufenuron,

Malathion, Mecarbam, Mervinphos, Mesulfenphos, Metaldehyd,. Methacrifos, Methamidophos, Methidathion, Methiocarb, Methomyl, Metolcarb, Milbemectin, Monocrotophos, Moxidectin,

Naled, NC 184, NI 25, Nitenpyram,

Omethoat, Oxamyl, Oxydemethon M, Oxydeprofos,

Parathion A, Parathion M, Permethrin, Phenthoat, Phorat, Phosalon, Phosmet, Phosphamidon, Phoxim, Pirimicarb, Pirimiphos M, Pirimiphos A, Profenofos, Promecarb, Propaphos, Propoxur, Prothiofos, Prothoat, Pymetrozin, Pyrachlophos, Pyradaphenthion, Pyresmethrin, Pyrethrum, Pyridaben, Pyrimidifen, Pyriproxifen,

Quinalphos,

RH 5992,

Salithion, Sebufos, Silafluofen, Sulfotep, Sulprofos,

Tebufenozid, Tebufenpyrad, Tebupirimphos, Teflubenzuron, Tefluthrin, Temephos, Terbam, Terbufos, Tetrachlorvinphos, Thiafenox, Thiodicarb, Thiofanox, Thiomethon, Thionazin, Thuringiensin, Tralomethrin, Triarathen, Triazophos, Triazuron, Trichlorfon, Triflumuron, Trimethacarb,

Vamidothion, XMC, Xylylcarb, YI 5301 / 5302, Zetamethrin.

Auch eine Mischung mit anderen bekannten Wirkstoffen, wie Herbiziden oder mit Düngemitteln und Wachstumsregulatoren ist möglich.

Die erfindungsgemäßen Wirkstoffe können ferner in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne daß der zugesetzte Synergist selbst aktiv wirksam sein muß.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 95 Gew.-% Wirkstoff, vorzugsweise zwischen 0,0001 und 1 Gew.-% liegen.

Die Anwendung geschieht in einer den Anwendungsformen angepaßten üblichen Weise.

Bei der Anwendung gegen Hygiene- und Vorratsschädlinge zeichnet sich der Wirkstoff durch eine hervorragende Residualwirkung auf Holz und Ton sowie durch eine gute Alkalistabilität auf gekälkten Unterlagen aus.

Die erfindungsgemäßen Wirkstoffe wirken nicht nur gegen Pflanzen-, Hygieneund Vorratsschädlinge, sondern auch auf dem veterinärmedizinischen Sektor gegen tierische Parasiten (Ektoparasiten) wie Schildzecken, Lederzekken, Räudemilben, Laufmilben, Fliegen (stechend und leckend), parasitierende Fliegenlarven, Läuse, Haarlinge, Federlinge und Flöhe. Zu diesen Parasiten gehören:

Aus der Ordnung der Anoplurida z.B. Haematopinus spp., Linognathus spp., Pediculus spp., Phtirus spp., Solenopotes spp..

Aus der Ordnung der Mallophagida und den Unterordnungen Amblycerina sowie Ischnocerina z.B. Trimenopon spp., Menopon spp., Trinoton spp., Bovicola spp., Werneckiella spp., Lepikentron spp., Damalina spp., Trichodectes spp., Felicola spp..

Aus der Ordnung Diptera und den Unterordnungen Nematocerina sowie Brachycerina z.B. Aedes spp., Anopheles spp., Culex spp., Simulium spp., Eusimulium spp., Phlebotomus spp., Lutzomyia spp., Culicoides spp., Chrysops spp., Hybomitra spp., Atylotus spp., Tabanus spp., Haematopota spp., Philipomyia spp., Braula spp., Musca spp.,

Hydrotaea spp., Stomoxys spp., Haematobia spp., Morellia spp., Fannia spp., Glossina spp., Calliphora spp., Lucilia spp., Chrysomyia spp., Wohlfahrtia spp., Sarcophaga spp., Oestrus spp., Hypoderma spp., Gasterophilus spp., Hippobosca spp., Lipoptena spp., Melophagus spp..

Aus der Ordnung der Siphonapterida z.B. Pulex spp., Ctenocephalides spp., Xenopsylla spp., Ceratophyllus spp..

Aus der Ordnung der Heteropterida z.B. Cimex spp., Triatoma spp., Rhodnius spp., Panstrongylus spp..

Aus der Ordnung der Blattarida z.B. Blatta orientalis, Periplaneta americana, Blattela germanica, Supella spp..

Aus der Unterklasse der Acaria (Acarida) und den Ordnungen der Meta- sowie Mesostigmata z.B. Argas spp., Ornithodorus spp., Otobius spp., Ixodes spp., Amblyomma spp., Boophilus spp., Dermacentor spp., Haemophysalis spp., Hyalomma spp., Rhipicephalus spp., Dermanyssus spp., Raillietia spp., Pneumonyssus spp., Sternostoma spp., Varroa spp..

Aus der Ordnung der Actinedida (Prostigmata) und Acaridida (Astigmata) z.B. Acarapis spp., Cheyletiella spp., Ornithocheyletia spp., Myobia spp., Psorergates spp., Demodex spp., Trombicula spp., Listrophorus spp., Acarus spp., Tyrophagus spp., Caloglyphus spp., Hypodectes spp., Pterolichus spp., Psoroptes spp., Chorioptes spp., Otodectes spp., Sarcoptes spp., Notoedres spp., Knemidocoptes spp., Cytodites spp., Laminosioptes spp..

Die erfindungsgemäßen Wirkstoffe der Formel (I) eignen sich auch zur Bekämpfung von Arthropoden, die landwirtschaftliche Nutztiere, wie z.B. Rinder, Schafe, Ziegen, Pferde, Schweine, Esel, Kamele, Büffel, Kaninchen, Hühner, Puten, Enten, Gänse, Bienen, sonstige Haustiere wie z.B. Hunde, Katzen, Stubenvögel, Aquarienfische sowie sogenannte Versuchstiere, wie z.B. Hamster, Meerschweinchen, Ratten und Mäuse befallen. Durch die Bekämpfung dieser Arthropoden sollen Todesfälle und Leistungsminderungen (bei Fleisch, Milch, Wolle, Häuten, Eiern, Honig usw.) vermindert werden, so daß durch den Einsatz der erfindungsgemäßen Wirkstoffe eine wirtschaftlichere und einfachere Tierhaltung möglich ist.

Die Anwendung der erfindungsgemäßen Wirkstoffe geschieht im Veterinärsektor in bekannter Weise durch enterale Verabreichung in Form von beispielsweise Tabletten,. Kapseln, Tränken, Drenchen, Granulaten, Pasten, Boli, des feed-through-Verfahrens, von Zäpfchen, durch parenterale Verabreichung, wie zum Beispiel durch Injektionen (intramuskulär, subcutan, intravenös, intraperitonal u.a.), Implantate, durch nasale Applikation, durch dermale Anwendung in Form beispielsweise des Tauchens oder Badens (Dippen), Sprühens (Spray), Aufgießens (Pour-on und Spot-on), des Waschens, des Einpuderns sowie mit Hilfe von wirkstoffhaltigen Formkörpern, wie Halsbändern, Ohrmarken, Schwanzmarken, Gliedmaßenbändern, Halftern, Markierungsvorrichtungen usw.

Bei der Anwendung für Vieh, Geflügel, Haustiere etc. kann man die Wirkstoffe der Formel (I) als Formulierungen (beispielsweise Pulver, Emulsionen, fließfähige Mittel), die die Wirkstoffe in einer Menge von 1 bis 80 Gew.-% enthalten, direkt oder nach 100 bis 10 000-facher Verdünnung anwenden oder sie als chemisches Bad verwenden.

Außerdem wurde gefunden, daß die erfindungsgemäßen Verbindungen der Formel (I) eine hohe insektizide Wirkung gegen Insekten zeigen, die technische Materialien zerstören.

Beispielhaft und vorzugsweise - ohne jedoch zu limitieren - seien die folgenden Insekten genannt:

Käfer wie Hylotrupes bajulus, Chlorophorus pilosis, Anobium punctatum, Xestobium rufovillosum, Ptilinus pecticornis, Dendrobium pertinex, Ernobius mollis, Priobium carpini, Lyctus brunneus, Lyctus africanus, Lyctus planicollis, Lyctus linearis, Lyctus pubescens, Trogoxylon aequale, Minthes rugicollis; Xyleborus spec. Tryptodendron spec. Apate monachus, Bostrychus capucins, Heterobostrychus brunneus, Sinoxylon spec. Dinoderus minutus;

Hautflügler wie Sirex juvencus, Urocerus gigas, Urocerus gigas taignus, Urocerus augur;

Termiten wie Kalotermes flavicollis, Cryptotermes brevis, Heterotermes indicola, Reticulitermes flavipes, Reticulitermes santonensis, Reticulitermes lucifugus, Mastotermes darwiniensis, Zootermopsis nevadensis, Coptotermes formosanus;

Borstenschwänze, wie Lepisma saccarina.

Unter technischen Materialien sind im vorliegenden Zusammenhang nicht-lebende Materialien zu verstehen, wie vorzugsweise Kunststoffe, Klebstoffe, Leime, Papiere und Kartone, Leder, Holz und Holzverarbeitungsprodukte und Anstrichmittel.

Ganz besonders bevorzugt handelt es sich bei dem vor Insektenbefall zu schützenden Material um Holz und Holzverarbeitungsprodukte.

Unter Holz und Holzverarbeitungsprodukten, welche durch das erfindungsgemäße Mittel bzw. dieses enthaltende Mischungen geschützt werden kann, ist beispielhaft zu verstehen: Bauholz, Holzbalken, Eisenbahnschwellen, Brückenteile, Bootsstege, Holzfahrzeuge, Kisten, Paletten, Container, Telefonmasten, Holzverkleidungen, Holzfenster und- türen, Sperrholz, Spanplatten, Tischlerarbeiten oder Holzprodukte, die ganz allgemein beim Hausbau oder in der

Bautischlerei Verwendung finden.

Die Wirkstoffe können als solche, in Form von Konzentraten oder allgemein üblichen Formulierungen wie Pulver, Granulate, Lösungen, Suspensionen, Emulsionen oder Pasten angewendet werden.

Die genannten Formulierungen können in bekannter Weise hergestellt werden, z.B. durch Vermischen der Wirkstoffe mit mindestens einem Lösungs- bzw. Verdünnungsmittel, Emulgator, Dispergier- und/oder Binde- oder Fixiermittels, Wasser-Repellent, gegebenenfalls Sikkative und UV-Stabilisatoren und gegebenenfalls Farbstoffen und Pigmenten sowie weiteren Verarbeitungshilfsmitteln.

Die zum Schutz von Holz und Holzwerkstoffen verwendeten insektiziden Mittel oder Konzentrate enthalten den erfindungsgemäßen Wirkstoff in einer Konzentration von 0,0001 bis 95 Gew.-%, insbesondere 0,001 bis 60 Gew.-%.

Die Menge der eingesetzten Mittel bzw. Konzentrate ist von der Art und dem Vorkommen der Insekten und von dem Medium abhängig. Die optimale Einsatzmenge kann bei der Anwendung jeweils durch Testreihen ermittelt werden. Im allgemeinen ist es jedoch ausreichend 0,0001 bis 20 Gew.-%, vorzugsweise 0,001 bis 10 Gew.-%, des Wirkstoffs, bezogen auf das zu schützende Material, einzusetzen.

Als Lösungs- und/oder Verdünnungsmittel dient ein organisch-chemisches Lösungsmittel oder Lösungsmittelgemisch und/oder ein öliges oder ölartiges schwer flüchtiges organisch-chemisches Lösungsmittel oder Lösungsmittelgemisch und/ der ein polares organisch-chemisches Lösungsmittel oder Lösungsmittelgemisch und/oder Wasser und gegebenenfalls einen Emulgator und/oder Netzmittel.

Als organisch-chemische Lösungsmittel werden vorzugsweise ölige oder ölartige Lösungsmittel mit einer Verdunstungszahl über 35 und einem Flammpunkt oberhalb 30°C, vorzugsweise oberhalb 45°C, eingesetzt. Als derartige schwerflüchtige, wasserunlösliche, ölige und ölartige Lösungsmittel werden entsprechende Mineralöle oder deren Aromatenfraktionen oder mineralölhaltige Lösungsmittelgemische, vorzugsweise Testbenzin, Petroleum und/oder Alkylbenzol verwendet.

Vorteilhaft gelangen Mineralöle mit einem Siedebereich von 170 bis 220°C, Testbenzin mit einem Siedebereich von 170 bis 220°C, Spindelöl mit einem Siedebereich von 250 bis 350°C, Petroleum bzw. Aromaten vom Siedebereich von 160 bis 280°C, Terpentinöl und dgl. zum Einsatz.

In einer bevorzugten Ausführungsform werden flüssige aliphatische Kohlenwasserstoffe mit einem Siedebereich von 180 bis 210°C oder hochsiedende Gemische von aromatischen und aliphatischen Kohlenwasserstoffen mit einem Siedebereich von 180 bis 220°C und/oder Spindeöl und/oder Monochlornaphthalin, vorzugsweise $\alpha$-Monochlornaphthalin, verwendet.

Die organischen schwerflüchtigen öligen oder ölartigen Lösungsmittel mit einer Verdunstungszahl über 35 und einem Flammpunkt oberhalb 30°C, vorzugsweise oberhalb 45°C, können teilweise durch leicht oder mittelflüchtige organisch-chemische Lösungsmittel ersetzt werden, mit der Maßgabe, daß das Lösungsmittelgemisch ebenfalls eine Verdunstungszahl über 35 und einen Flammpunkt oberhalb 30°C, vorzugsweise oberhalb 45°C, aufweist und daß das Insektizid-Fungizid-Gemisch in diesem Lösungsmittelgemisch löslich oder emulgierbar ist.

Nach einer bevorzugten Ausführungsform wird ein Teil des organisch-chemischen Lösungsmittel oder Lösungsmittelgemisches oder ein aliphatisches polares organisch-chemisches Lösungsmittel oder Lösungsmittelgemisch ersetzt. Vorzugsweise gelangen Hydroxyl- und/oder Ester- und/oder Ethergruppen enthaltende aliphatische organisch-chemische Lösungsmittel wie beispielsweise Glycolether, Ester oder dgl. zur Anwendung.

Als organisch-chemische Bindemittel werden im Rahmen der vorliegenden Erfindung die an sich bekannten wasserverdünnbaren und/oder in den eingesetzten organisch-chemischen Lösungsmitteln löslichen oder dispergier- bzw. emulgierbaren Kunstharze und/oder bindende trocknende Öle, insbesondere Bindemittel bestehend aus oder enthaltend ein Acrylatharz, ein Vinylharz, z.B. Polyvinylacetat, Polyesterharz, Polykondensations- oder Polyadditionsharz, Polyurethanharz, Alkydharz bzw. modifiziertes Alkydharz, Phenolharz, Kohlenwasserstoffharz wie Inden-Cumaronharz, Siliconharz, trocknende pflanzliche und/oder trocknende Öle und/oder physikalisch trocknende Bindemittel auf der Basis eines Natur- und/oder Kunstharzes verwendet.

Das als Bindemittel verwendete Kunstharz kann in Form einer Emulsion, Dispersion oder Lösung, eingesetzt werden. Als Bindemittel können auch Bitumen oder bituminöse Substanzen bis zu 10 Gew.-%, verwendet werden. Zusätzlich können bekannte Farbstoffe, Pigmente, wasserabweisende Mittel, Geruchskorrigentien und Inhibitoren bzw. Korrosionsschutzmittel und dgl. eingesetzt werden.

Bevorzugt ist gemäß der Erfindung als organisch-chemische Bindemittel mindestens ein Alkydharz bzw. modifiziertes Alkydharz und/oder ein trocknendes pflanzliches Öl im Mittel oder im Konzentrat enthalten. Bevorzugt werden gemäß der Erfindung Alkydharze mit einem Ölgehalt von mehr als 45 Gew.-%, vorzugsweise 50 bis 68 Gew.-%, verwendet.

Das erwähnte Bindemittel kann ganz oder teilweise durch ein Fixierungsmittel(gemisch) oder durch ein(en) Weichmacher(gemisch) ersetzt werden. Diese Zusätze sollen einer Verflüchtigung der Wirkstoffe sowie einer Kristallisation bzw. Ausfällem vorbeugen. Vorzugsweise ersetzen sie 0,01 bis 30 % des Bindemittels (bezogen auf 100 % des eingesetzten Bindemittels).

Die Weichmacher stammen aus den chemischen Klassen der Phthalsäureester wie Dibutyl-, Dioctyl- oder Benzylbutylphthalat, Phosphorsäureester wie Tributylphosphat, Adipinsäureester wie Di-(2-ethylhexyl)-adipat, Stearate wie Butylstearat oder Amylstearat, Oleate wie Butyloleat, Glycerinether oder höhermolekulare Glykolether, Glycerinester sowie p-Toluolsulfonsäureester.

Fixierungsmittel basieren chemisch auf Polyvinylalkylethern wie z.B. Polyvinylmethylether oder Ketonen wie Benzophenon, Ethylenbenzophenon.

Als Lösungs- bzw. Verdünnungsmittel kommt insbesondere auch Wasser in Frage, gegebenenfalls in Mischung mit einem oder mehreren der oben genannten organisch-chemischen Lösungs- bzw. Verdünnungsmittel, Emulgatoren und Dispergatoren.

Ein besonders effektiver Holzschutz wird durch großtechnische Imprägnierverfahren, z.B. Vakuum, Doppelvakuum oder Druckverfahren, erzielt.

Die anwendungsfertigen Mittel können gegebenenfalls noch weitere Insektizide und gegebenenfalls noch ein oder mehrere Fungizide enthalten.

Als zusätzliche Zumischpartner kommen vorzugsweise die in der WO 94/29 268 genannten Insektizide und Fungizide in Frage. Die in diesem Dokument genannten Verbindungen sind ausdrücklicher Bestandteil der vorliegenden Anmeldung.

Als ganz besonders bevorzugte Zumischpartner können Insektizide, wie Chlorpyriphos, Phoxim, Silafluofin, Alphamethrin, Cyfluthrin, Cypermethrin, Deltamethrin, Permethrin, Imidacloprid, NI-25, Flufenoxuron, Hexaflumuron und Triflumuron,
sowie Fungizide wie Epoxyconazole, Hexaconazole, Azaconazole, Propiconazole, Tebuconazole, Cyproconazole, Metconazole, Imazalil, Dichlorfluanid, Tolylfluanid, 3-Iod-2-propinyl-butylcarbamat, N-Octyl-isothiazolin-3-on und 4,5-Dichlor-N-octylisothiazolin-3-on, sein.

Die Wirkstoffe können in die üblichen Formulierungen übergeführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage: z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z. B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgierund/oder schaumerzeugende Mittel kommen in Frage: z.B. nicht-ionogene und anionische Emulgatoren, wie Polyoxyethylen-Fettsäure-Ester, Pölyoxyethylen-Fettalkohol-Ether, z.B. Alkylarylpolyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide in Frage, beispielsweise Anilide, wie z.B. Diflufenican und Propanil; Arylcarbonsäuren, wie z.B. Dichlorpicolinsäure, Dicamba und Picloram; Aryloxyalkansäuren, wie z.B. 2,4 D,

2,4 DB, 2,4 DP, Fluroxypyr, MCPA, MCPP und Triclopyr; Aryloxy-phenoxy-alkansäureester, wie z.B. Diclofop-methyl, Fenoxaprop-ethyl, Fluazifop-butyl, Haloxyfop-methyl und Quizalofop-ethyl; Azinone, wie z.B. Chloridazon und Norflurazon; Carbamate, wie z.B. Chlorpropham, Desmedipham, Phenmedipham und Propham; Chloracetanilide, wie z. B. Alachlor, Acetochlor, Butachlor, Metazachlor, Metolachlor, Pretilachlor und Propachlor; Dinitroaniline, wie z.B. Oryzalin, Pendimethalin und Trifluralin; Diphenylether, wie z.B. Acifluorfen, Bifenox, Fluoroglycofen, Fomesafen, Halosafen, Lactofen und Oxyfluorfen; Harnstoffe, wie z.B. Chlortoluron, Diuron, Fluometuron, Isoproturon, Linuron und Methabenzthiazuron; Hydroxylamine, wie z.B. Alloxydim, Clethodim, Cycloxydim, Sethoxydim und Tralkoxydim; Imidazolinone, wie z.B. Imazethapyr, Imazamethabenz, Imazapyr und Imazaquin; Nitrile, wie z.B. Bromoxynil, Dichlobenil und Ioxynil; Oxyacetamide, wie z.B. Mefenacet; Sulfonylharnstoffe, wie z.B. Amidosulfuron, Bensulfuron-methyl, Chlorimuron-ethyl, Chlorsulfuron, Cinosulfuron, Metsulfuronmethyl, Nicosulfuron, Primisulfuron, Pyrazosulfuron-ethyl, Thifensulfuron-methyl, Triasulfuron und Tribenuron-methyl; Thiolcarbamate, wie z.B. Butylate, Cycloate, Diallate, EPTC, Esprocarb, Molinate, Prosulfocarb, Thiobencarb und Triallate; Triazine, wie z.B. Atrazin, Cyanazin, Simazin, Simetryne, Terbutryne und Terbutylazin; Triazinone, wie z.B. Hexazinon, Metamitron und Metribuzin; Sonstige, wie z.B. Aminotriazol, Benfuresate, Bentazone, Cinmethylin, Clomazone, Clopyralid, Difenzoquat, Dithiopyr, Ethofumesate, Fluorochloridone, Glufosinate, Glyphosate, Isoxaben, Pyridate, Quinchlorac, Quinmerac, Sulphosate und Tridiphane.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden. Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 1 g und 10 kg Wirkstoff pro Hektar Bodenfläche, vorzugsweise zwischen 5 g und 5 kg pro ha.

Die Herstellung und die Verwendung der erfindungsgemäßen Wirkstoffe geht aus den nachfolgenden Beispielen hervor.

## Herstellungsbeispiele:

### Beispiel 1

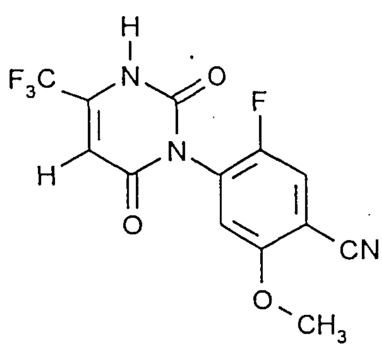

(Verfahren (a))

Eine Mischung aus 3,8 g (12 mMol) 1-(4-Cyano-2,5-difluor-phenyl)-3,6-dihydro-2,6-dioxo-4-trifluormethyl-1(2H)-pyrimidin, 1,5 g (21 mMol) Natriummethylat und 50 ml N-Methyl-pyrrolidon wird 36 Stunden bei 130°C gerührt. Nach Abkühlen der Mischung und Verdünnen mit Essigsäureethylester auf etwa das dreifache Volumen wird mit Wasser gewaschen und mittels Chromatographie über eine Kieselgelsäule (Cyclohexan/Essigsäureethylester, Vol.: 1/1) das Produkt isoliert.

Man erhält 1,6 g (41% der Theorie) 1-(4-Cyano-2-fluor-5-methoxy-phenyl)-3,6-dihydro-2,6-dioxo-4-trifluormethyl-1(2H)-pyrimidin vom Schmelzpunkt 114°C.

**Beispiel 2**

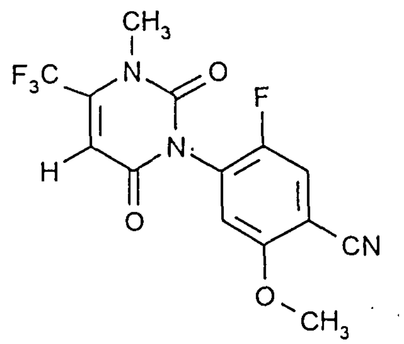

(Verfahren (b))

Eine Mischung aus 1,5 g (4 mMol) 1-(4-Cyano-2-fluor-5-methoxy-phenyl)-3,6-dihydro-2,6-dioxo-4-trifluormethyl-1(2H)-pyrimidin, 0,6 g (5 mMol) Dimethylsulfat, 0,7 g (5 mMol) Kaliumcarbonat und 100 ml Acetonitril wird 18 Stunden unter Rückfluß erhitzt und dann im Wasserstrahlvakuum eingeengt. Der Rückstand wird mit Wasser/Essigsäureethylester geschüttelt, die organische Phase abgetrennt, mit Natriumsulfat getrocknet und filtriert. Das Filtrat wird im Wasserstrahlvakuum eingeengt, der Rückstand mit Essigsäureethylester/Diisopropylether digeriert und das hierbei kristallin angefallene Produkt durch Absaugen isoliert.

Man erhält 1,0 g (73% der Theorie) 1-(4-Cyano-2-fluor-5-methoxy-phenyl)-3,6-dihydro-2,6-dioxo-3-methyl-4-trifluormethyl-1(2H)-pyrimidin vom Schmelzpunkt 155°C.

Analog zu den Beispielen 1 und 2 sowie entsprechend der allgemeinen Beschreibung der erfindungsgemäßen Herstellungsverfahren können beispielsweise auch die in der nachstehenden Tabelle 1 aufgeführten Verbindungen der Formel (I) hergestellt werden.

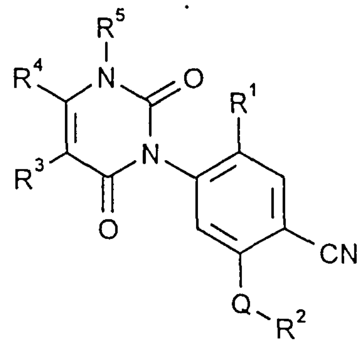

**Tabelle 1:** Beispiele für die Verbindungen der Formel (I)

| Bsp.-Nr. | Q | $R^1$ | $R^2$ | $R^3$ | $R^4$ | $R^5$ | Schmelz-punkt (°C) |
|---|---|---|---|---|---|---|---|
| 3 | S | H | $CH_2C_6H_5$ | H | $CF_3$ | $CH_3$ | 88 |
| 4 | O | F | $CH_2C_6H_5$ | H | $CF_3$ | $CH_3$ | 45 |
| 5 | S | F | 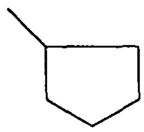 | H | $CF_3$ | $CH_3$ | ($n_D^{20}$ = 1,5449) |
| 6 | S | F | 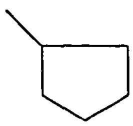 | H | $CF_3$ | H | 79 |

| Bsp.-Nr. | Q | $R^1$ | $R^2$ | $R^3$ | $R^4$ | $R^5$ | Schmelzpunkt (°C) |
|---|---|---|---|---|---|---|---|
| 7 | S | F | 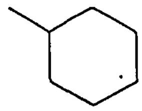 | H | $CF_3$ | $CH_3$ | |
| 8 | S | F | 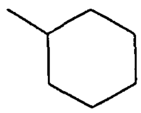 | $CH_3$ | $CF_3$ | $CH_3$ | |
| 9 | S | F | 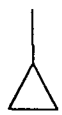 | H | $CF_3$ | $CH_3$ | |
| 10 | S | F | 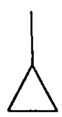 | $CH_3$ | $CF_3$ | $CH_3$ | |
| 11 | S | F | 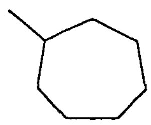 | H | $CF_3$ | $CH_3$ | |
| 12 | S | F | 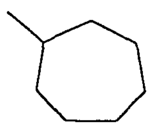 | $CH_3$ | $CF_3$ | $CH_3$ | |

| Bsp.-Nr. | Q | $R^1$ | $R^2$ | $R^3$ | $R^4$ | $R^5$ | Schmelzpunkt (°C) |
|---|---|---|---|---|---|---|---|
| 13 | S | F | 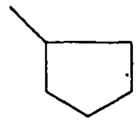 | H | $CF_3$ | $(CH_2)_3F$ | $n_D^{20}$ = 1,5438 |
| 14 | S | F | 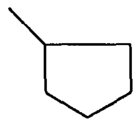 | $CH_3$ | $CF_3$ | $(CH_2)_3F$ | |
| 15 | S | F | 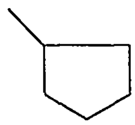 | H | $CH_3$ | $(CH_2)_3F$ | |
| 16 | S | F | 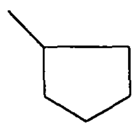 | $CH_3$ | $CH_3$ | $(CH_2)_3F$ | |
| 17 | O | F | 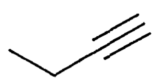 | H | $CF_3$ | $CH_3$ | 145 |
| 18 | O | F | 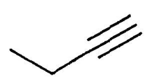 | H | $CF_3$ | $C_2H_5$ | |
| 19 | O | F | 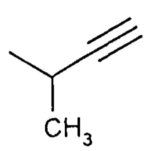 | H | $CF_3$ | $CH_3$ | 115 |

| Bsp.-Nr. | Q | $R^1$ | $R^2$ | $R^3$ | $R^4$ | $R^5$ | Schmelz-punkt (°C) |
|---|---|---|---|---|---|---|---|
| 20 | O | F | 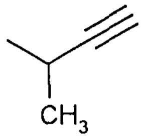 | H | $CF_3$ | $C_2H_5$ | |
| 21 | O | H | 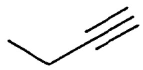 | H | $CF_3$ | $CH_3$ | |
| 22 | O | H | 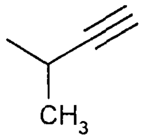 | H | $CF_3$ | $CH_3$ | |
| 23 | O | F | 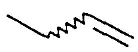 | H | $CF_3$ | $CH_3$ | 157 |
| 24 | O | F | 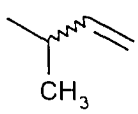 | H | $CF_3$ | $CH_3$ | |
| 25 | O | F | 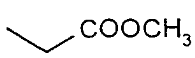 | H | $CF_3$ | $CH_3$ | >200 |
| 26 | O | F | 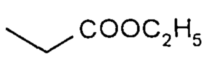 | H | $CF_3$ | $CH_3$ | |
| 27 | O | F | 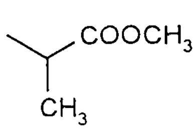 | H | $CF_3$ | $CH_3$ | |

| Bsp.-Nr. | Q | $R^1$ | $R^2$ | $R^3$ | $R^4$ | $R^5$ | Schmelzpunkt (°C) |
|---|---|---|---|---|---|---|---|
| 28 | O | F | 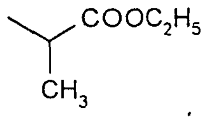 | H | $CF_3$ | $CH_3$ | |
| 29 | O | F | 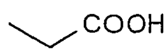 | H | $CF_3$ | $CH_3$ | |
| 30 | O | F | 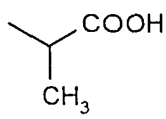 | H | $CF_3$ | $CH_3$ | |
| 31 | O | F | $C_2H_5$ | H | $CF_3$ | H | 123 |
| 32 | O | F | $C_2H_5$ | H | $CF_3$ | $CH_3$ | 121 |
| 33 | O | F | $CH_3$ | H | $CF_3$ | $C_2H_5$ | 90 |
| 34 | O | F | 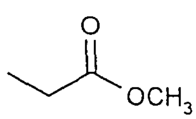 | H | $CF_3$ | H | 185 |
| 35 | O | F | 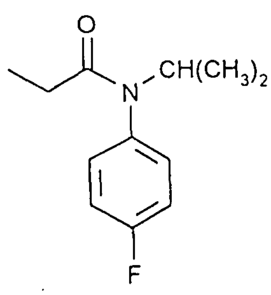 | H | $CF_3$ | $CH_3$ | >200 |

| Bsp.-Nr. | Q | $R^1$ | $R^2$ | $R^3$ | $R^4$ | $R^5$ | Schmelz-punkt (°C) |
|---|---|---|---|---|---|---|---|
| 36 | O | F | 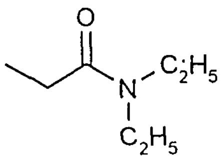 | H | $CF_3$ | $CH_3$ | 1H-NMR ($CDCl_3$, δ): 6,33 ppm (s, 1H) |
| 37 | O | F | 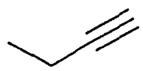 | H | $CF_3$ | H | 140 |
| 38 | O | F | 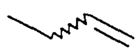 | H | $CF_3$ | H | |
| 39 | O | F | 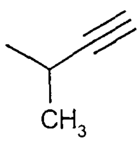 | H | $CF_3$ | H | 202 |
| 40 | O | F | $C_2H_5$ | H | $CF_3$ | $CHF_2$ | 177 |
| 41 | O | F | 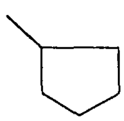 | H | $CF_3$ | H | 200 |
| 42 | O | F | 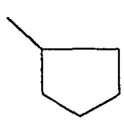 | H | $CF_3$ | $CH_3$ | |

| Bsp.-Nr. | Q | $R^1$ | $R^2$ | $R^3$ | $R^4$ | $R^5$ | Schmelzpunkt (°C) |
|---|---|---|---|---|---|---|---|
| 43 | O | F | 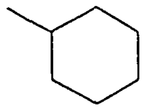 | H | $CF_3$ | H | 221 |
| 44 | O | F | 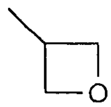 | H | $CF_3$ | H | 130 |
| 45 | O | F | 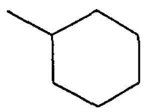 | H | $CF_3$ | $CH_3$ | |
| 46 | O | F | 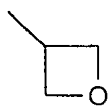 | H | $CF_3$ | $CH_3$ | |
| 47 | S | F | 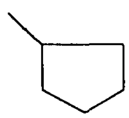 | $CH_3$ | $CF_3$ | $CH_3$ | $n_D^{20}$ = 1,5628 |
| 48 | O | F | 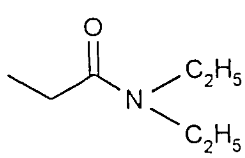 | H | $CF_3$ | H | 88 |

**Anwendungsbeispiele:**

**Beispiel A**

Pre-emergence-Test

| | |
|---|---|
| Lösungsmittel | 5 Gewichtsteile Aceton |
| Emulgator | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät. Der Boden wird nach 24 Stunden mit der Wirkstoffzubereitung begossen bzw. gespritzt. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit.

Nach drei Wochen wird der Schädigungsgrad der Pflanzen bonitiert in % Schädigung im Vergleich zur Entwicklung der unbehandelten Kontrolle.

Es bedeuten:

0 % = keine Wirkung (wie unbehandelte Kontrolle)

100 % = totale Vernichtung

In diesem Test zeigen bei Aufwandmengen zwischen 8 und 125 g/ha beispielsweise die Verbindungen gemäß Herstellungsbeispiele 2 und 4 bei guter Verträglichkeit gegenüber Kulturpflanzen, wie z.B. Weizen (0 %), sehr starke Wirkung gegen Unkräuter wie Digitaria (90-100 %), Setaria (95-100 %), Abutilon (100 %), Chenopodium (100 %) und Matricaria (100 %).

**Beispiel B**

Post-emergence-Test

| | |
|---|---|
| Lösungsmittel | 5 Gewichtsteile Aceton |
| Emulgator | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5-15 cm haben so, daß die jeweils gewünschten Wirkstoffmengen pro Flächeneinheit ausgebracht werden. Die Konzentration der Spritzbrühe wird so gewählt, daß in 2000 1 Wasser/ha die jeweils gewünschten Wirkstoffmengen ausgebracht werden.

Nach drei Wochen wird der Schädigungsgrad der Pflanzen bonitiert in % Schädigung im Vergleich zur Entwicklung der unbehandelten Kontrolle.

Es bedeuten:

0 % = keine Wirkung (wie unbehandelte Kontrolle)

100 % = totale Vernichtung

In diesem Test zeigen beispielsweise die Verbindungen gemäß Herstellungsbeispiele 2 und 4 bei Aufwandmengen zwischen 30 und 500 g/ha starke Wirkung gegen Unkräuter wie Panicum (95-100 %), Setaria (90-100 %), Sorghum (80-95 %), Abutilon (100 %), Chenopodium (100 %) sowie Matricaria (100 %).

**Beispiel C**

Phaedon-Larven-Test

| | |
|---|---|
| Lösungsmittel | 7 Gewichtsteile Dimethylformamid |
| Emulgator | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.
Kohlblätter (Brassica oleracea) werden durch Tauchen in die Wirkstoffzubereitung der gewünschten Konzentration behandelt und mit Meerrettichblattkäfer-Larven (Phaedon cochleariae) besetzt, solange die Blätter noch feucht sind.
Nach der gewünschten Zeit wird die Abtötung in % bestimmt. Dabei bedeutet 100 %, daß alle Käfer-Larven abgetötet wurden; 0 % bedeutet, daß keine Käfer-Larven abgetötet wurden.
In diesem Test bewirkt z.B. die Verbindung des Herstellungsbeispiels 4 bei einer beispielhaften Wirkstoffkonzentration von 0,1 % eine Abtötung von 100 % nach 7 Tagen.

**Beispiel D**

Plutella-Test

| | |
|---|---|
| Lösungsmittel | 7 Gewichtsteile Dimethylfomamid |
| Emulgator | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.
Kohlblätter (Brassica oleracea) werden durch Tauchen in die Wirkstoffzubereitung der gewünschten Konzentration behandelt und mit Raupen der Kohlschabe (Plutella maculipennis) besetzt, solange die Blätter noch feucht sind.
Nach der gewünschten Zeit wird die Abtötung in % bestimmt. Dabei bedeutet 100 %, daß alle Raupen abgetötet wurden; 0 % bedeutet, daß keine Raupen abgetötet wurden.
In diesem Test bewirkt z.B. die Verbindung des Herstellungsbeispiels 4 bei einer beispielhaften Wirkstoffkonzentration von 0,1 % eine Abtötung von 100 % nach 7 Tagen.

**Patentansprüche**

1. Cyanophenyluracile der allgemeinen Formel (I)

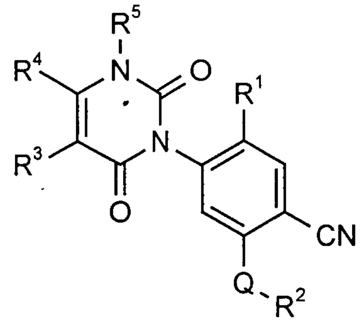

(I)

in welcher

Q für O, S, SO oder $SO_2$ steht,

$R^1$ für Wasserstoff, Cyano, Fluor oder Chlor steht,

$R^2$ für jeweils gegebenenfalls durch Cyano, Carboxy, Carbamoyl, Thiocarbamoyl, Fluor, Chlor, Brom, durch $C_1$-$C_4$-Alkyl, $C_1$-$C_4$-Alkoxy, $C_1$-$C_4$-Alkylthio, $C_1$-$C_4$-Alkylsulfinyl, $C_1$-$C_4$-Alkylsulfonyl, $C_1$-$C_4$-Alkylcarbonyl oder $C_1$-$C_4$-Alkoxy-carbonyl (welches jeweils gegebenenfalls durch Fluor und/oder Chlor sind), durch Phenyl, Phenoxy oder Phenylthio (welche jeweils gegebenenfalls durch Fluor, Chlor, Brom, Cyano, Methyl, Methoxy, Trifluormethyl und/oder Trifluormethoxy substituiert sind) substituiertes Furyl, Tetrahydrofuryl, Thienyl, Tetrahydrothienyl, Oxetanyl, Thietanyl, Oxazolyl, Isoxazolyl, Thiazolyl, Oxadiazolyl, Thiadiazolyl, Pyrazolyl, Pyridinyl, Pyrimidinyl, Triazinyl, Indolyl, Chinolinyl oder Chinoxalinyl steht,

$R^3$ für Wasserstoff, Fluor, Chlor, Brom oder für jeweils gegebenenfalls durch Fluor und/oder Chlor substituiertes Alkyl oder Alkoxy mit jeweils 1 bis 4 Kohlenstoffatomen steht,

$R^4$ für gegebenenfalls durch Fluor und/oder Chlor substituiertes Alkyl mit 1 bis 4 Kohlenstoffatomen steht und

$R^5$ für Wasserstoff oder für jeweils gegebenenfalls durch Fluor, Chlor oder $C_1$-$C_4$-Alkoxy substituiertes Alkyl, Alkoxy, Alkenyl oder Alkinyl mit jeweils bis zu 6 Kohlenstoffatomen steht.

**2.** Cyanophenyluracile der allgemeinen Formel (I) gemäß Anspruch 1, **dadurch gekennzeichnet, daß**

Q für O, S, SO oder $SO_2$ steht,

$R^1$ für Wasserstoff, Fluor oder Chlor steht,

$R^2$ für jeweils gegebenenfalls durch Cyano, Carboxy, Carbamoyl, Thiocarbamoyl, Fluor, Chlor, Brom, Methyl, Ethyl, n- oder i-Propyl, Methoxy, Ethoxy, n- oder i-Propoxy, Methylthio, Ethylthio, Methylsulfinyl, Ethylsulfinyl, Methylsulfonyl, Ethylsulfonyl, Acetyl, Propionyl, Methoxycarbonyl, Ethoxycarbonyl, n- oder i-Propoxycarbonyl, durch Phenyl, Phenoxy oder Phenylthio substituiertes substituiertes Furyl, Tetrahydrofuryl, Thienyl, Tetrahydrothienyl, Oxetanyl, Thietanyl, Oxazolyl, Isoxazolyl, Thiazolyl, Oxadiazolyl, Thiadiazolyl, Pyrazolyl, Pyridinyl, Pyrimidinyl, Triazinyl, Indolyl, Chinolinyl oder Chinoxalinyl steht,

$R^3$ für Wasserstoff, Fluor, Chlor, Brom oder Methyl steht,

$R^4$ für Methyl, Ethyl, Difluormethyl, Dichlormethyl, Trifluormethyl, Trichlormethyl, Chlordifluormethyl, Fluordichlormethyl, Chlorethyl, Fluorethyl, Dichlorethyl, Dichlorethyl, Chlorfluorethyl, Chlordifluorethyl, Fluordichlorethyl, Trifluorethyl, Tetrafluorethyl, Chlortrifluorethyl oder Pentafluorethyl steht und

$R^5$ für Wasserstoff oder für jeweils gegebenenfalls durch Fluor, Chlor, Methoxy oder Ethoxy substituiertes Methyl, Ethyl, n- oder i-Propyl, n-, i-, s- oder t-Butyl, Methoxy, Ethoxy, n- oder i-Propoxy, n-, i- oder s-Butoxy, Propenyl, Butenyl, Propinyl oder Butinyl steht.

**3.** Cyanophenyluracile der allgemeinen Formel (Ia)

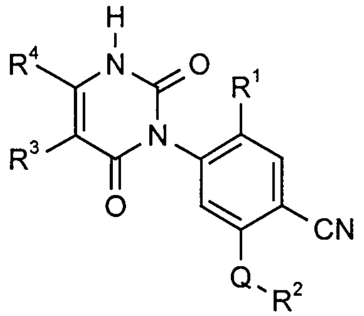

(Ia)

**dadurch gekennzeichnet, daß**
Q, $R^1$, $R^2$, $R^3$ und $R^4$ die in Anspruch 1 angegebenen Bedeutungen haben.

**4.** Verfahren zur Herstellung von Cyanophenyluracilen der allgemeinen Formel (I)

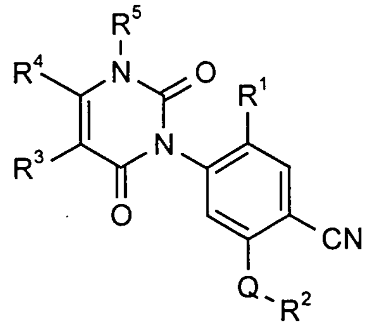

(I)

in welcher
$R^1$, $R^2$, $R^3$, $R^4$, $R^5$ und Q die in Anspruch 1 genannten Bedeutungen haben,
**dadurch gekennzeichnet, daß** man

(a) substituierte Halogenphenyluracile der allgemeinen Formel (II)

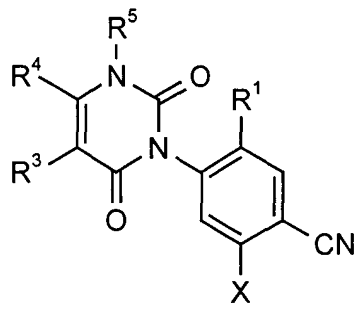

(II)

in welcher

| | |
|---|---|
| $R^1$, $R^3$, $R^4$ und $R^5$ | die oben angegebenen Bedeutungen haben und |
| X | für Halogen steht, |

mit nucleophilen Verbindungen der allgemeinen Formel (III)

$$M\text{-}Q\text{-}R^2 \quad \text{(III)}$$

in welcher

Q und $R^2$ die oben angegebenen Bedeutungen haben und

M für Wasserstoff oder ein Alkalimetall steht,

gegebenenfalls in Gegenwart eines Reaktionshilfsmittels und gegebenenfalls in Gegenwart eines Verdünnungsmittels umsetzt,
oder daß man

(b) substituierte Cyanophenyluracile der allgemeinen Formel (Ia)

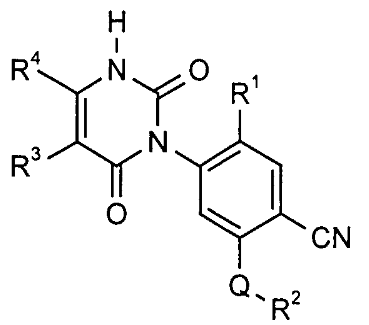

(Ia)

in welcher
Q, $R^1$, $R^2$, $R^3$ und $R^4$ die oben angegebenen Bedeutungen haben,
mit einem Alkylierungsmittel gegebenenfalls in Gegenwart eines Reaktionshilfsmittels und gegebenenfalls in Gegenwart eines Verdünnungsmittels umsetzt.

**5.** Herbizide Mittel, **gekennzeichnet durch** einen Gehalt an mindestens einem Cyanophenyluracil der allgemeinen Formel (I) gemäß einem der Ansprüche 1 bis 3.

**6.** Verfahren zur Bekämpfung von unerwünschten Pflanzen, **dadurch gekennzeichnet, daß** man Cyanophenyluracile der allgemeinen Formel (I) gemäß einem der Ansprüche 1 bis 3 auf unerwünschte Pflanzen und/oder ihre Lebensräume einwirken läßt.

**7.** Verwendung von Cyanophenyluracilen der allgemeinen Formel (I) gemäß einem der Ansprüche 1 bis 3 zur Bekämpfung von unerwünschten Pflanzen.

**8.** Verfahren zur Herstellung von herbiziden Mitteln, **dadurch gekennzeichnet, daß** man Cyanophenyluracile der allgemeinen Formel (I) gemäß einem der Ansprüche 1 bis 3 mit Streckmitteln und/oder oberflächenaktiven Substanzen vermischt.

Europäisches Patentamt

# EUROPÄISCHER RECHERCHENBERICHT

Nummer der Anmeldung
EP 02 00 9276

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.7) |
|---|---|---|---|
| A | EP 0 260 621 A (HOFFMAN LA ROCHE)<br>23. März 1988 (1988-03-23)<br>* das ganze Dokument *<br>----- | 1-8 | C07D239/54<br>C07D405/12<br>A01N43/54 |
| | | | RECHERCHIERTE SACHGEBIETE (Int.Cl.7) |
| | | | C07D<br>A01N |

Der vorliegende Recherchenbericht wurde für alle Patentansprüche erstellt

| Recherchenort | Abschlußdatum der Recherche | Prüfer |
|---|---|---|
| DEN HAAG | 27. Mai 2002 | Francois, J |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie,übereinstimmendes Dokument

EPO FORM 1503 03.82 (P04C03)

## ANHANG ZUM EUROPÄISCHEN RECHERCHENBERICHT ÜBER DIE EUROPÄISCHE PATENTANMELDUNG NR. EP 02 00 9276

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

27-05-2002

| Im Recherchenbericht angeführtes Patentdokument | Datum der Veröffentlichung | Mitglied(er) der Patentfamilie | Datum der Veröffentlichung |
|---|---|---|---|
| EP 260621 A | 23-03-1988 | AU 7849087 A | 31-03-1988 |
| | | CN 87106833 A | 27-04-1988 |
| | | EP 0260621 A2 | 23-03-1988 |
| | | HU 44904 A2 | 30-05-1988 |
| | | US 4812164 A | 14-03-1989 |
| | | BR 8704796 A | 17-05-1988 |
| | | JP 63107967 A | 12-05-1988 |
| | | ZA 8706839 A | 18-03-1988 |

EPO FORM P0461

Für nähere Einzelheiten zu diesem Anhang : siehe Amtsblatt des Europäischen Patentamts, Nr.12/82